# EUROPEAN PATENT APPLICATION

(11) **EP 4 818 206 A1**
(43) Date of publication of application: **07.10.2026**
(21) Application number: 24896717.6
(22) Date of filing: 29.11.2024
(51) Int. Cl.: C07K 16/28, C12N 15/13, A61K 39/395, A61P 35/00

(54) **ANTI-CD84 ANTIBODY AND USE THEREOF**

(30) Priority: 01.12.2023 CN 202311640467
(71) Applicant: Shanghai Symray Biopharma Inc., Shanghai 201109 (CN)
(72) Inventor: WU, Dang, Shanghai 201109 (CN); SUN, Zhen, Shanghai 201109 (CN); LI, Yanan, Shanghai 201109 (CN); QIU, Yangsheng, Shanghai 201109 (CN); ZHAO, Wenxiang, Shanghai 201109 (CN)
(74) Representative: Cabinet Becker et Associés
(86) International application number: PCT/CN2024/135649
(87) International publication number: WO 2025/113635

(57) **Abstract**

Provided are an antibody binding to a CD84 antigen, or an antigen-binding fragment thereof, a nucleic acid molecule encoding the specific antibody and the antigen-binding fragment thereof, a vector comprising the nucleic acid molecule, a host cell comprising the vector, a chimeric antigen receptor or immunoconjugate comprising the antibody and the antigen-binding fragment thereof, and a use of them in the preparation of a drug for treating or preventing diseases, especially for treating hematologic tumors, and an application of them in detection of products.

## Description

### TECHNICAL FIELD

The present invention relates to anti-CD84 antibodies, preparation methods thereof, compositions thereof and uses thereof. The present invention also provides methods of treating CD84-related diseases and/or conditions, such as cancer.

### BACKGROUND

CD84 (also known as LY9B or SLAMF5) is a membrane glycoprotein belonging to members of signaling lymphocytic activation molecule (SLAM) family, which *per se* is a subset of the larger CD2 cell surface receptor subgroup within the Ig superfamily. It self-associates to form homophilic dimers, and is mainly expressed on hematopoietic cells.

CD84 is widely expressed in most immune cell subsets and functions as a homophilic adhesion molecule, the signaling via which can activate or inhibit leukocyte functions, depending on cell types and the activation or differentiation stage thereof. CD84-mediated signaling regulates a variety of immune processes, including T cell cytokine secretion, natural killer cell cytotoxicity, monocyte activation, autophagy, cognate T:B interactions, and B-cell tolerance at germinal center checkpoints. Recent studies have found that CD84 is associated with autoimmune diseases and lymphoid tissue hyperplasia. CD84 is widely expressed in various hematological malignancies, including acute myeloid leukemia (AML), chronic lymphocytic leukemia (CLL), blastic plasmacytoid dendritic cell neoplasm (BPDCN), chronic myeloid leukemia (CML), myelodysplastic syndrome (MDS), acute lymphoblastic leukemia (ALL), multiple myeloma (MM), hairy cell leukemia (HCL), or Hodgkin's lymphoma (HL). Relevant studies have shown that inhibition of CD function has therapeutic effects on hematological malignancies, particularly AML.

However, there are few studies on antibody therapy targeting CD84 for the treatment of AML at present. Therefore, the development of drugs targeting CD84 for the treatment of AML has important clinical value.

### SUMMARY OF THE INVENTION

The present invention provides novel anti-CD84 antibodies. The CD84 antibody molecules of the present invention exhibit high affinity for both human and monkey CD84; and all the antibody molecules of the present invention specifically recognize CD84, do not bind to other proteins of the same family, and exhibit potent killing activity against tumor cells, thereby achieving the purpose of treating hematological malignancies.

Therefore, in one aspect, the present invention provides anti-CD84 antibodies or antigen-binding fragments thereof, comprising a heavy chain variable region HCDR1 selected from SEQ ID NOs: 1, 8, 13 and 22, and/or a heavy chain variable region HCDR2 selected from SEQ ID NOs: 2, 9, 14, 18 and 23, and/or a heavy chain variable region HCDR3 selected from SEQ ID NOs: 3, 10, 15, 21 and 24, and/or a light chain variable region LCDR1 selected from SEQ ID NOs: 4, 6, 11, 16, 19, 25 and 27, and/or a light chain variable region LCDR2 selected from NAK, FAS, WAS and YAS, and/or a light chain variable region LCDR3 selected from SEQ ID NOs: 5, 7, 12, 17, 20 and 26, or variants of combinations of the CDR sequences.

In some embodiments, the present invention also provides anti-CD84 antibodies or antigen-binding fragments thereof. The antibodies bind to the same or overlapping epitope as and/or competes for binding to CD84 with exemplary antibodies of the present invention (such as antibodies having the VH and VL sequence combinations of antibodies listed in Table A below), and/or inhibits (e.g., competitively inhibits) the exemplary antibodies of the present invention.

In some embodiments, the antibodies are bispecific antibodies, which further comprise an antigen-binding fragment that binds to a second antigen, specifically binding to CD3, and the second antigen-binding fragment comprises a VH comprising the amino acid sequence set forth in SEQ ID NO: 61, and a VL comprising the amino acid sequence set forth in SEQ ID NO: 62.

In some embodiments, the present invention provides nucleic acids encoding the antibodies or antigen-binding fragments thereof of the present invention, vectors comprising the nucleic acids, and host cells comprising the vectors.

In some embodiments, the present invention provides methods for preparing the antibodies or antigen-binding fragments thereof of the present invention.

In some embodiments, the present invention provides chimeric antigen receptors, immunoconjugates or pharmaceutical combinations comprising the antibodies of the present invention.

In some embodiments, the present invention also provides methods of and uses for preventing or treating CD84-related diseases using the antibodies or antigen-binding fragments thereof of the present invention, wherein the diseases include, but are not limited to tumors, such as hematological malignancies, further including acute myeloid leukemia (AML), chronic lymphocytic leukemia (CLL), blastic plasmacytoid dendritic cell neoplasm (BPDCN), chronic myeloid leukemia (CML), myelodysplastic syndrome (MDS), acute lymphoblastic leukemia (ALL), multiple myeloma (MM), hairy cell leukemia (HCL), or Hodgkin's lymphoma (HL).

In some embodiments, the antibodies of the present invention may be used as the sole active agent, or may be administered in combination with other therapies or therapeutic agents.

In some embodiments, the present invention also provides methods and kits for detecting CD84 in a sample using the antibodies or antigen-binding fragments thereof of the present invention.

The present invention is further illustrated in the following drawings and specific embodiments. However, these drawings and specific embodiments should not be considered as limiting the scope of the present invention, and changes readily conceived by the skilled in the art shall be included within the spirit of the present invention and the scope of protection of the appended claims.

### BRIEF DESCRIPTION OF DRAWINGS

Fig. 1 shows the construction of CD84-overexpressing cell lines.
Fig. 2 shows the CDR sequences of the light/heavy chains of the chimeric antibodies.
Fig. 3 shows the affinity assay of the chimeric antibodies with 239T-hCD84 cells.
Fig. 4 shows the VH/VL sequence information of the humanized antibodies.
Fig. 5 shows the affinity assay of the humanized antibodies with 293T-hCD84 cells.
Fig. 6 shows the affinity assay of the humanized antibodies with HEL cells.
Fig. 7 shows the affinity assay of the humanized antibodies with U937 cells.
Fig. 8 shows a schematic diagram of the structure of the CD84×CD3 bispecific antibodies.
Fig. 9 shows the CD84×CD3 bispecific antibody-mediated toxicity of PBMCs against U937 cells.
Fig. 10 shows the affinity assay of the CD84×CD3 bispecific antibodies with CD84 and CD3 proteins.
Fig. 11 shows the affinity assay of the CD84×CD3 bispecific antibodies with different cells.
Fig. 12 shows the cytotoxicity assay of the CD84×CD3 bispecific antibodies against tumor cells.
Fig. 13 shows the *in vitro* safety evaluation assay of the CD84×CD3 bispecific antibodies.
Fig. 14 shows the kinetic assay of the CD84×CD3 bispecific antibodies with CD84 protein.
Fig. 15 shows the kinetic assay of the CD84×CD3 bispecific antibodies with CD3 protein.
Fig. 16 shows the *in vivo* anti-tumor assay of the CD84×CD3 bispecific antibodies.

### DETAILED DESCRIPTION OF THE INVENTION

### Definition

The terminology used herein is for the purpose of describing embodiments only and is not intended to be limiting. Unless otherwise defined, all technical and scientific terms used herein have the same meaning as commonly understood by one of ordinary skill in the art to which this disclosure belongs.

The term "comprising" or "including" means including the elements, integers or steps, but does not exclude any other elements, integers or steps. When the term "comprising" or "including" is used herein, unless otherwise indicated, combinations of the stated elements, integers or steps are also encompassed. For example, when referring to an antibody variable region "comprising" a specific sequence, it is also intended to encompass an antibody variable region consisting of that specific sequence.

The term "and/or", such as "X and/or Y", should be understood to mean "X and Y" or "X or Y" and should be used to provide explicit support for both meanings or either meaning.

The term "antibody" is used herein in the broadest sense to refer to a protein comprising an antigen-binding site, and encompasses natural and artificial antibodies of various structures, including but not limited to monoclonal antibodies, polyclonal antibodies, multispecific antibodies (e.g., bispecific antibodies), single-chain variable fragment, intact antibodies, and antibody fragments.

The term "whole antibody" (which may be used interchangeably herein with "full-length antibody", "complete antibody" and "intact antibody") comprises at least two heavy chains (H) and two light chains (L). Each heavy chain consists of a heavy chain variable region (abbreviated herein as VH) and a heavy chain constant region. The heavy chain constant region consists of three domains CH1, CH2 and CH3. Each light chain consists of a light chain variable region (abbreviated herein as VL) and a light chain constant region. The light chain constant region consists of one domain CL. The variable regions are domains in the heavy or light chains of an antibody that are involved in binding of the antibody to its antigen. The constant regions are not directly involved in binding of the antibody to the antigen, but exhibit various effector functions. The light chains of antibodies can be classified into one of two types (designated as kappa (κ) and lambda (λ)) based on the amino acid sequences of their constant domains. The heavy chains of antibodies can be divided into five major distinct types depending on the amino acid sequences of their heavy chain constant regions: IgA, IgD, IgE, IgG and IgM, and several of these types can be further divided into subclasses, such as IgG1, IgG2, IgG3 and IgG4, IgA1 and IgA2. The heavy chain constant regions corresponding to the different antibody types are referred to as α, δ, ε, γ and µ, respectively. The term "isotype" refers to an antibody type determined by the heavy chain constant regions of the antibody. See, e.g., Fundamental Immunology, Ch.7 (Paul, W., ed., 2nd ed., Raven Press, N.Y. (1989)).

The term "CD84" refers to an expressed isoform of the CD84 gene. Examples include, but are not limited to, Q9UIB8-1, Q9UIB8-2, Q9UIB8-3, Q9UIB8-4, Q9UIB8-5, Q9UIB8-6 and Q9UIB8-7. An exemplary amino acid sequence of CD84 involved in the present invention is shown below:

MAQHHLWILLLCLQTWPEAAGKDSEIFTVNGILGESVTFPVNIQEPRQVKIIAWTSKTSVAY
VTPGDSETAPWTVTHRNYYERIHALGPNYNLVISDLRMEDAGDYKADINTQADPYTTTKR
YNLQIYRRLGKPKITQSLMASVNSTCNVTLTCSVEKEEKNVTYNWSPLGEEGNVLQIFQTP
EDQELTYTCTAQNPVSNNSDSISARQLCADIAMGFRTHHTGLLSVLAMFFLLVLILSSVFLFR
LFKRRQGRIFPEGSCLNTFTKNPYAASKKTIYTYIMASRNTQPAESRIYDEILQSKVLPSKEEP
VNTVYSEVQFADKMGKASTQDSKPPGTSSYEIVI (SEQ ID NO:60).

The term "CD3" refers to the human CD3 protein multi-subunit complex. The CD3 protein multi-subunit complex is composed of six distinct polypeptide chains. Unless otherwise stated, the term "CD3" includes any CD3 variants, isoforms, and species homologs that are naturally expressed by cells (including T cells) or can be expressed on cells transfected with genes or cDNA encoding those polypeptides.

The term "bispecific antibody" in the context of the present invention should be understood as an antibody having two different antigen-binding fragments defined by different antibody sequences. This may be understood as binding to different targets, but also includes binding to different epitopes of one target. The term "bispecific antibody" as used herein should be understood in its broadest sense, including full-length bispecific antibodies and antigen-binding fragments thereof. Bispecific antibodies may contain additional modifications, such as non-naturally occurring amino acids, mutations in the Fc region, and mutations in glycosylation sites. Bispecific antibodies also include post-translational modified antibodies, fusion proteins containing the antigenic determinants of antibodies, and immunoglobulin molecules containing any other modifications to the antigen recognition site, as long as these antibodies exhibit the desired biological activity.

The term "antigen-binding fragment" (which may be used interchangeably herein with "antibody fragment" and "antigen-binding portion") refers to a molecule that is not an intact antibody, which comprises a portion in an intact antibody used for binding to the antigen bound by the intact antibody. As understood by the skilled in the art, the antigen-binding portion of an antibody typically comprises amino acid residues from "complementarity-determining regions" or "CDRs". Antigen-binding fragments may be prepared by recombinant DNA techniques, or by enzymatic or chemical cleavage of intact antibodies. Antigen-binding fragments include, but are not limited to, Fab, scFab, Fab', F(ab')2, Fab'-SH, Fv, single-chain Fv, diabody, triabody, tetrabody, minibody, and single-domain antibody (sdAb).

The term "scFv" refers to a fusion protein comprising at least one antibody fragment comprising the variable region of a light chain and at least one antibody fragment comprising the variable region of a heavy chain, wherein the light chain and heavy chain variable regions are contiguous (e.g., via a synthetic linker such as a short flexible polypeptide linker) and capable of being expressed as a single-chain polypeptide, and wherein the scFv retains the specificity of the intact antibody from which it is derived. Unless specified, as used herein, the scFv may have the VL and VH variable regions in any order (e.g., relative to the N-terminus and C-terminus of the polypeptide), and the scFv may comprise VL-linker-VH or VH-linker-VL.

The term "chimeric antibody" refers to an antibody in which the variable region sequences are derived from one species and the constant region sequences are derived from another species, for example, an antibody in which the variable region sequences are derived from a mouse antibody and the constant region sequences are derived from a human antibody.

The term "humanized antibody" refers to an antibody in which CDR sequences derived from other mammalian species, such as mouse germline, are grafted onto human framework sequences. Additional framework region modifications may be made within the human framework sequences, and/or additional amino acid modifications may be made in the CDR sequences, for example, to perform affinity maturation of the antibody.

The term "isolated" antibody is an antibody that has been isolated from components in its natural environment. In some embodiments, the antibody is purified to a purity of greater than 95% or 99%, which is determined by, for example, electrophoresis (e.g., SDS-PAGE, isoelectric focusing (IEF), capillary electrophoresis) or chromatography (e.g., ion exchange or reverse-phase HPLC). For a review of methods for assessing antibody purity, see, e.g., Flatman, S. et al., J. Chrom. B 848(2007)79-87.

The term "chimeric antigen receptor" or alternatively "CAR" refers to a group of polypeptides, usually two groups in the simplest embodiment, which, when present in immune effector cells, provide the cells with specificity against target cells (usually cancer cells) and generate intracellular signals. In certain embodiments, the CAR comprises at least one extracellular binding region, a transmembrane region, and an intracellular signaling region. In certain aspects, polypeptide groups are contiguous to each other.

The term "epitope" refers to the region of an antigen to which an antibody binds. An epitope can be formed by contiguous amino acids or by non-contiguous amino acids juxtaposed by tertiary folding of a protein.

The term "Fc region" is used herein to define the C-terminal region of an immunoglobulin heavy chain containing at least a portion of the constant region. The term includes native sequence Fc-regions and variant Fc-regions. In one embodiment, the human IgG heavy chain Fc-region extends from Cys226 or from Pro230 of the heavy chain to the carboxyl-terminus. However, the C-terminal lysine (Lys447) of the Fc-region may be present or absent. Unless otherwise indicated herein, amino acid residues in the Fc-region or constant region are numbered according to the EU numbering system, also known as the EU index, as described in Kabat, E.A. et al., Sequences of Proteins of Immunological Interest, 5th ed., Public Health Service, National Institutes of Health, Bethesda, MD (1991), NIH Publication 91-3242.

As used herein, the term sequence "identity" refers to the extent to which two (nucleotide or amino acid) sequences have identical residues at the same positions in an alignment, and is usually expressed as a percentage. Preferably, identity is determined over the entire length of the sequences being compared. Thus, two copies having completely identical sequences have 100% identity. The skilled in the art knows that several algorithms can be used to determine sequence identity, such as Blast (Altschul et al. (1997) Nucleic Acids Res. 25: 3389-3402), Blast2 (Altschul et al. (1990) J. Mol. Biol. 215: 403-410), Smith-Waterman (Smith et al. (1981) J. Mol. Biol. 147: 195-197), and ClustalW.

In the present invention, the term "conservative modification" refers to amino acid modifications that do not significantly affect or alter the binding characteristics of the antibody or antibody fragment containing the amino acid sequence. These conservative modifications include conservative substitutions, additions, and deletions of amino acids. Modifications can be introduced into the chimeric antigen receptors of the present invention by standard techniques known in the art, such as site-directed mutagenesis and PCR-mediated mutagenesis. A conservative amino acid substitution is a substitution in which an amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine). Conservative modifications may be selected, for example, based on similarity in polarity, charge, solubility, hydrophobicity, hydrophilicity, and/or the amphipathic nature of the residues involved.

Various aspects of the present invention will be further described in detail in the following subsections.

### i. Anti-CD84 Antibodies of the Present Invention

In one aspect, the present invention provides antibodies or antigen-binding fragments thereof, particularly humanized antibodies or antigen-binding fragments thereof, that specifically bind to CD84, preferably human CD84 protein. In some embodiments, the antigen-binding fragments of the antibodies in the present invention are antibody fragments selected from Fab, Fab', Fab'-SH, Fv, single-chain antibody such as scFv, (Fab')2 fragment, single-domain antibody, diabody (dAb), and linear antibody.

### Antibody CDR Regions

"Complementarity-determining region" or "CDR region" or "CDR" (which may be used interchangeably herein with hypervariable region "HVR") is the amino acid region in an antibody variable region primarily responsible for binding to an antigenic epitope. The CDRs of the heavy and light chains are commonly referred to as CDR1, CDR2, and CDR3, numbered sequentially starting from the N-terminus. The CDRs located within the heavy chain variable domain of an antibody are referred to as HCDR1, HCDR2, and HCDR3, while the CDRs located within the light chain variable domain of an antibody are referred to as LCDR1, LCDR2, and LCDR3.

**Table A: VH and VL Sequence Combinations of Exemplary Antibodies**

| Antibody | VH comprising or consisting of the amino acid sequence set forth in SEQ ID NO below | VL comprising or consisting of the amino acid sequence set forth in SEQ ID NO below |
|---|---|---|
| 1 | SEQ ID NO: 30 | SEQ ID NO: 29 |
| 2 | SEQ ID NO: 43 | SEQ ID NO: 38 |
| 3 | SEQ ID NO: 45 | SEQ ID NO: 46 |
| 4 | SEQ ID NO: 53 | SEQ ID NO: 54 |

It is well known to the skilled in the art that CDRs of an antibody can be defined by a variety of methods in the art, for example, Chothia (Chothia et al. (1989) Nature 342: 877-883; Al-Lazikani et al., "Standard conformations for the canonical structures of immunoglobulins", Journal of Molecular Biology, 273, 927-948 (1997)) based on the three-dimensional structure of the antibody and the topology of the CDR loops; Kabat (Kabat et al., Sequences of Proteins of Immunological Interest, 4th ed., U.S. Department of Health and Human Services, National Institutes of Health (1987)) based on antibody sequence variability; AbM (University of Bath); Contact (University College London); the international ImMunoGeneTics database (IMGT) (worldwide web imgt.cines.fr/); and the North CDR definition based on affinity propagation clustering using a large number of crystal structures. It should be understood by the skilled in the art that, unless otherwise specified, the terms "CDR" and "complementarity-determining region" of a given antibody or region thereof (e.g., variable region) should be understood to encompass complementarity-determining regions defined by any of the above known schemes as described in the present invention. The corresponding CDRs for various numbering systems are well known to the skilled in the art, as shown in Table B:

**Table B: Common CDR Definition Schemes**

| | Kabat | AbM | Chothia | Contact | IMGT |
|---|---|---|---|---|---|
| LCDR1 | L24-L34 | L24-L34 | L24-L34 | L30-L36 | L27-L32 |
| LCDR2 | L50-L56 | L50-L56 | L50-L56 | L46-L55 | L50-L51 |
| LCDR3 | L89-L97 | L89-L97 | L89-L97 | L89-L96 | L89-L97 |
| HCDR1/VHH-CDR1 | H31-H35B | H26-H35B | H26-H32 | H30-H35B | H26-H35B |
| HCDR2/VHH-CDR2 | H50-H65 | H50-H58 | H52-H56 | H47-H58 | H51-H56 |
| HCDR3/VHH-CDR3 | H95-H102 | H95-H102 | H95-H102 | H93-H101 | H93-H102 |

Unless otherwise specified, the terms "CDR" or "CDR sequence" or "HVR" or "HVR sequence" in the present invention encompass HVR or CDR sequences determined by any of the above methods.

Unless otherwise specified, the "IMGT" numbering rule applies to all variable region and CDR sequences in the Examples of the present disclosure.

In a preferred embodiment, CDRs of the antibodies of the present invention are shown in Table C, and are preferably CDR sequences defined by the extended Kabat and IMGT CDR numbering schemes.

**Table C. Exemplary CDR Sequence Combinations of the Present Invention**

| Combination | HCDR1 comprising or consisting of the amino acid sequence set forth in SEQ ID NO below | HCDR2 comprising or consisting of the amino acid sequence set forth in SEQ ID NO below | HCDR3 comprising or consisting of the amino acid sequence set forth in SEQ ID NO below | LCDR1 comprising or consisting of the amino acid sequence set forth in SEQ ID NO below | LCDR2 comprising or consisting of the amino acid sequence set forth below | LCDR3 comprising or consisting of the amino acid sequence set forth in SEQ ID NO below |
|---|---|---|---|---|---|---|
| 1 | SEQ ID NO: 1 | SEQ ID NO: 2 | SEQ ID NO: 3 | SEQ ID NO: 4 | NAK | SEQ ID NO: 5 |
| 2 | SEQ ID NO: 1 | SEQ ID NO: 2 | SEQ ID NO: 3 | SEQ ID NO: 6 | FAS | SEQ ID NO: 7 |
| 3 | SEQ ID NO: 8 | SEQ ID NO: 9 | SEQ ID NO: 10 | SEQ ID NO: 11 | WAS | SEQ ID NO: 12 |
| 4 | SEQ ID NO: 13 | SEQ ID NO: 14 | SEQ ID NO: 15 | SEQ ID NO: 16 | YAS | SEQ ID NO: 17 |
| 5 | SEQ ID NO: 8 | SEQ ID NO: 18 | SEQ ID NO: 10 | SEQ ID NO: 19 | WAS | SEQ ID NO: 20 |
| 6 | SEQ ID NO: 8 | SEQ ID NO: 9 | SEQ ID NO: 21 | SEQ ID NO: 11 | WAS | SEQ ID NO: 12 |
| 7 | SEQ ID NO: 22 | SEQ ID NO: 23 | SEQ ID NO: 24 | SEQ ID NO: 25 | WAS | SEQ ID NO: 26 |
| 8 | SEQ ID NO: 22 | SEQ ID NO: 23 | SEQ ID NO: 24 | SEQ ID NO: 27 | WAS | SEQ ID NO: 26 |

In one embodiment, the antibodies or the antigen-binding fragments thereof of the present invention comprise a heavy chain variable region and a light chain variable region, wherein the antibodies comprise:
(i) six CDR sequences contained within the VH and VL sequences of any antibody listed in Table A; or
(ii) six CDR sequences in any combination listed in Table C; or
(iii) six CDR sequences comprising at least one and not more than 10, or 5, 4, 3, 2, 1 amino acid alterations (preferably amino acid substitutions, preferably conservative substitutions) in the six CDR regions, relative to the sequences of (i) or (ii).

In one embodiment, the antibodies or antigen-binding fragments thereof of the present invention comprises three complementarity-determining regions of the heavy chain variable region (HCDRs) and three complementarity-determining regions of the light chain variable region (LCDRs), wherein:
(i) HCDR1 comprises or consists of the amino acid sequence of SEQ ID NO: 1, HCDR2 comprises or consists of the amino acid sequence of SEQ ID NO: 2, HCDR3 comprises or consists of the amino acid sequence of SEQ ID NO: 3, LCDR1 comprises or consists of the amino acid sequence of SEQ ID NO: 4, LCDR2 comprises or consists of NAK, and LCDR3 comprises or consists of the amino acid sequence of SEQ ID NO: 5; or
(ii) HCDR1 comprises or consists of the amino acid sequence of SEQ ID NO: 1, HCDR2 comprises or consists of the amino acid sequence of SEQ ID NO: 2, HCDR3 comprises or consists of the amino acid sequence of SEQ ID NO: 3, LCDR1 comprises or consists of the amino acid sequence of SEQ ID NO: 6, LCDR2 comprises or consists of FAS, and LCDR3 comprises or consists of the amino acid sequence of SEQ ID NO: 7; or
(iii) HCDR1 comprises or consists of the amino acid sequence of SEQ ID NO: 8, HCDR2 comprises or consists of the amino acid sequence of SEQ ID NO: 9, HCDR3 comprises or consists of the amino acid sequence of SEQ ID NO: 10, LCDR1 comprises or consists of the amino acid sequence of SEQ ID NO: 11, LCDR2 comprises or consists of WAS, and LCDR3 comprises or consists of the amino acid sequence of SEQ ID NO: 12; or
(iv) HCDR1 comprises or consists of the amino acid sequence of SEQ ID NO: 13, HCDR2 comprises or consists of the amino acid sequence of SEQ ID NO: 14, HCDR3 comprises or consists of the amino acid sequence of SEQ ID NO: 15, LCDR1 comprises or consists of the amino acid sequence of SEQ ID NO: 16, LCDR2 comprises or consists of YAS, and LCDR3 comprises or consists of the amino acid sequence of SEQ ID NO: 17; or
(v) HCDR1 comprises or consists of the amino acid sequence of SEQ ID NO: 8, HCDR2 comprises or consists of the amino acid sequence of SEQ ID NO: 18, HCDR3 comprises or consists of the amino acid sequence of SEQ ID NO: 10, LCDR1 comprises or consists of the amino acid sequence of SEQ ID NO: 19, LCDR2 comprises or consists of WAS, and LCDR3 comprises or consists of the amino acid sequence of SEQ ID NO: 20; or
(vi) HCDR1 comprises or consists of the amino acid sequence of SEQ ID NO: 8, HCDR2 comprises or consists of the amino acid sequence of SEQ ID NO: 9, HCDR3 comprises or consists of the amino acid sequence of SEQ ID NO: 21, LCDR1 comprises or consists of the amino acid sequence of SEQ ID NO: 11, LCDR2 comprises or consists of WAS, and LCDR3 comprises or consists of the amino acid sequence of SEQ ID NO: 12; or
(vii) HCDR1 comprises or consists of the amino acid sequence of SEQ ID NO: 22, HCDR2 comprises or consists of the amino acid sequence of SEQ ID NO: 23, HCDR3 comprises or consists of the amino acid sequence of SEQ ID NO: 24, LCDR1 comprises or consists of the amino acid sequence of SEQ ID NO: 25, LCDR2 comprises or consists of WAS, and LCDR3 comprises or consists of the amino acid sequence of SEQ ID NO: 26; or
(viii) HCDR1 comprises or consists of the amino acid sequence of SEQ ID NO: 22, HCDR2 comprises or consists of the amino acid sequence of SEQ ID NO: 23, HCDR3 comprises or consists of the amino acid sequence of SEQ ID NO: 24, LCDR1 comprises or consists of the amino acid sequence of SEQ ID NO: 27, LCDR2 comprises or consists of WAS, and LCDR3 comprises or consists of the amino acid sequence of SEQ ID NO: 26;
or wherein the antibodies comprise variants of one of the CDR sequence combinations (i) - (viii), wherein the variants comprise a total of at least one and not more than 5, 4, 3, 2 or 1 amino acid alteration(s) (preferably amino acid substitution(s), preferably conservative substitution(s)) in the six CDR regions, and preferably the heavy chain CDR3 remains unchanged; wherein amino acid X at position 5 in the amino acid sequence of SEQ ID NO: 9 is N, D, or Q.

In one embodiment, the antibodies or antigen-binding fragments thereof of the present invention comprise:
(i) the HCDR1, HCDR2 and HCDR3 sequences of the heavy chain variable region set forth in SEQ ID NO: 30, and the LCDR1, LCDR2 and LCDR3 sequences of the light chain variable region set forth in SEQ ID NO: 29; or
(ii) the HCDR1, HCDR2 and HCDR3 sequences of the heavy chain variable region set forth in SEQ ID NO: 43, and the LCDR1, LCDR2 and LCDR3 sequences of the light chain variable region set forth in SEQ ID NO: 38; or
(iii) the HCDR1, HCDR2 and HCDR3 sequences of the heavy chain variable region set forth in SEQ ID NO: 45, and the LCDR1, LCDR2 and LCDR3 sequences of the light chain variable region set forth in SEQ ID NO: 46; or
(iv) the HCDR1, HCDR2 and HCDR3 sequences of the heavy chain variable region set forth in SEQ ID NO: 53, and the LCDR1, LCDR2 and LCDR3 sequences of the light chain variable region set forth in SEQ ID NO: 54.

### Antibody Variable Regions

A "variable region" or "variable domain" is a domain in the heavy or light chain of an antibody that is involved in the binding of the antibody to its antigen. The heavy chain variable region (VH) and light chain variable region (VL) can be further subdivided into hypervariable regions (HVRs, also referred to as complementarity-determining regions (CDRs)), interspersed with more conserved regions (namely framework regions (FRs)). Each VH and VL consists of three CDRs and four FRs, arranged from the amino terminus to the carboxyl terminus in the following order: FR1, CDR1, FR2, CDR2, FR3, CDR3, FR4. In some cases, a single VH or VL domain is sufficient to confer antigen-binding specificity. In addition, an antibody that binds to a particular antigen can be isolated by screening a library of complementary VL or VH domains using the VH or VL domain from an antibody that binds to the antigen (see, e.g., Portolano, S. et al., J. Immunol. 150(1993)880-887; Clackson, T. et al., Nature 352(1991)624-628).

CDR grafting is another modification method of antibody variable regions known in the art. Since CDR sequences are responsible for most antibody-antigen interactions, recombinant antibody variants that mimic the properties of a known antibody can be constructed. In such antibody variants, CDR sequences from the known antibody are grafted onto the framework regions of a different antibody with distinct properties. Therefore, in one embodiment, the present invention relates to such anti-CD84 antibodies or antigen-binding fragments thereof, comprising CDR sequences of the heavy and light chain variable regions from one of the antibodies in Table A, but having different framework region sequences. Framework region sequences for substitution can be obtained from public DNA databases, including germline antibody gene sequences, or from CD84 antibody sequences reported in published literature. For example, germline DNAs encoding human heavy and light chains variable region genes can be obtained from the GenBank database. The antibody protein sequence can be compared with protein sequences in a database using a sequence similarity search tool, such as Gapped BLAST. Preferably, the framework sequences for substitution have structural similarity to the framework sequences of the antibodies of the present invention selected for modification, for example, a framework sequence having a sequence identity of at least 80%, 85%, 90%, or 95%, 96%, 97%, 98%, 99% or more. In some embodiments, humanization of the antibodies can be performed according to the method of Example 3.

Therefore, in one embodiment, the antibodies of the present invention comprise the heavy chain variable region VH sequence of any antibody listed in Table A, or consist of the amino acid sequences. In a further embodiment, the antibodies of the present invention comprise variants of the VH sequences.

In another embodiment, the antibodies of the present invention comprise the light chain variable region VL sequence of any antibody listed in Table A, or consist of the amino acid sequences. In a further embodiment, the antibodies of the present invention comprise variants of the VL sequences.

In one embodiment, the antibodies of the present invention comprise:
(i) a VH sequence comprising the amino acid sequence set forth in SEQ ID NO: 30 or a variant thereof, and/or a VL sequence comprising the amino acid sequence set forth in SEQ ID NO: 29 or a variant thereof; or
(ii) a VH sequence comprising the amino acid sequence set forth in SEQ ID NO: 43 or a variant thereof, and/or a VL sequence comprising the amino acid sequence set forth in SEQ ID NO: 38 or a variant thereof; or
(iii) a VH sequence comprising the amino acid sequence set forth in SEQ ID NO: 45 or a variant thereof, and/or a VL sequence comprising the amino acid sequence set forth in SEQ ID NO: 46 or a variant thereof; or
(iv) a VH sequence comprising the amino acid sequence set forth in SEQ ID NO: 53 or a variant thereof, and/or a VL sequence comprising the amino acid sequence set forth in SEQ ID NO: 54 or a variant thereof.

In one embodiment, a variant of the VH sequence has at least 80%, 85%, 90%, 92%, 95%, 97%, 98%, 99% or higher amino acid sequence identity to a reference VH sequence (preferably, in the full length or in the three regions CDR1, CDR2 and CDR3). In one embodiment, a variant of the VH sequence comprises at least one and not more than 30, 10, or 5, 4, 3, 2, 1, 0 amino acid alterations (preferably amino acid substitutions, preferably conservative substitutions) in amino acid sequence compared to a reference VH sequence (preferably, in the full length or in the three regions CDR1, CDR2 and CDR3). Preferably, the sequence differences do not occur in the CDR regions.

In a preferred embodiment, a variant of the VL sequence has at least 80%, 85%, 90%, 92%, 95%, 97%, 98%, 99% or higher amino acid sequence identity to a reference VL sequence (preferably, in the full length or in the three regions CDR1, CDR2 and CDR3). In a preferred embodiment, a variant of VL sequence comprises at least one and not more than 30, 10, or 5, 4, 3, 2, 1, 0 amino acid alterations (preferably amino acid substitutions, preferably conservative substitutions) in amino acid sequence compared to a reference VL sequence (preferably, in the full length or in the three regions CDR1, CDR2 and CDR3). Preferably, the sequence differences do not occur in the CDR regions.

In a preferred embodiment, the antibodies of the present invention comprise the heavy chain variable region and light chain variable region VH/VL sequence pair of any antibody listed in Table A, or consists of the amino acid sequence pair. The present invention also provides variants of the antibodies, for example, the variants having at least 95%-99% identity or comprising not more than 10 amino acid alterations in VH, VL, or VH and VL.

In any of the above embodiments, preferably, relative to a reference antibody, the heavy chain variable region of an antibody variant comprises not more than 10, preferably not more than 5 (e.g., 3, 2, 1 or 0) amino acid alterations (preferably amino acid substitutions, preferably conservative substitutions) in one or more CDR (preferably all three CDR) regions.

In any of the above embodiments, preferably, relative to a reference antibody, the light chain variable region VL of an antibody variant comprises not more than 10, preferably not more than 5 (e.g., 3, 2, 1 or 0) amino acid alterations (preferably amino acid substitutions, preferably conservative substitutions) in one or more CDR (preferably all three CDR) regions.

### Heavy and Light Chains of the Antibodies

In one embodiment, the antibodies of the present invention comprise a heavy chain constant region and/or a light chain constant region. In some embodiments, the antibodies of the present invention comprise a heavy chain Fc region, for example an Fc region of an IgG1, IgG2, IgG3 or IgG4 isotype. In other embodiments, the antibodies of the present invention comprise an IgG1-Fc region, particularly a human IgG1-Fc region. In some embodiments, the antibodies of the present invention comprise a κ light chain constant region, for example a human κ light chain constant region.

In a preferred embodiment, the antibodies of the present invention comprise an IgG1-Fc region variant, wherein the variant comprises an amino acid sequence with at least one, two or three, but not more than 20, 10 or 5 amino acid alterations, or an amino acid sequence having at least 80%, 85%, 90%, 92%, 95%, 97%, 98%, 99% or higher identity, compared to the corresponding reference sequence. Preferably, the amino acid alterations do not occur in the CDR regions, more preferably do not occur in the variable regions.

### CD84×CD3 Bispecific Antibodies

In some embodiments, the anti-CD84 antibodies of the present invention are bispecific antibodies, which further comprise a second antigen-binding fragment.

In some embodiments, the first or second antigen-binding fragments of the anti-CD84 bispecific antibodies of the present invention are selected from the following antibody fragments: Fab, Fab', Fab'-SH, Fv, single-chain antibody, scFv, (Fab')2 fragment, single-domain antibody, diabody (dAb), or linear antibody.

In some embodiments, the second antigen of the anti-CD84 antibodies of the present invention is a tumor-associated antigen or an immune cell antigen, preferably a T cell antigen, wherein the T cell antigen is selected from T cell receptor (TCR), CD3, CD4, CD8, CD16, CD25, CD28, CD38, CD44, CD62L, CD69, ICOS, 41-BB (CD137), and NKG2D.

In some embodiments, the anti-CD84 bispecific antibodies of the present invention comprise a first antigen-binding fragment that binds to CD84, comprising a first light chain variable region (VL1) and a first heavy chain variable region (VH1), and a second antigen-binding fragment that binds to CD3, comprising a second light chain variable region (VL2) and a second heavy chain variable region (VH2), wherein VL1 and VH1 comprise the CDRs of the antibodies shown in Table A or Table C above, and the VH2 comprises HCDRs 1-3 of the amino acid sequence set forth in SEQ ID NO: 61, and the VL2 comprises LCDRs 1-3 of the amino acid sequence set forth in SEQ ID NO: 62, respectively.

In some embodiments, the CD3 antigen-binding fragments of the anti-CD84 bispecific antibodies of the present invention comprise one VH domain and one VL domain selected from Table D.

**Table D CD3-arm VH/VL sequence information**

| VH (SEQ ID NO:61) | VL (SEQ ID NO:62) |
|---|---|
| EVQLVESGGGLVQPGGSLRLSCAASGFTFNTY AMNWVRQAPGKGLEWVARIRSKYNNYATYY AASVKGRFTISRDDSKNSLYLQMNSLKTEDT AVYYCARHGNFGDSYVSWFAYWGQGTLVTV SS | QTVVTQEPSLTVSPGGTVTLTCRSSTGAV TTSNYANWVQQKPGQAPRGLIGGTNKR APGTPARFSGSLLGGKAALTLSGVQPED EAEYYCALWYSNHWVFGGGTKLTVL |

The bispecific antibodies disclosed herein may comprise an Fc region comprising CH2 and CH3 of an antibody.

The Fc region may be any isotype, including but not limited to IgG1, IgG2, IgG3 and IgG4, and may comprise one or more mutations or modifications. In one embodiment, the Fc region is or derived from IgG1 isotype, optionally with one or more mutations or modifications. In another embodiment, the Fc region is or derived from IgG4 isotype, optionally with one or more mutations or modifications. In one embodiment, the Fc region is human IgG1 Fc.

In a specific embodiment of the present invention, the heavy chain variable region VH and light chain variable region VL in the second antigen-binding fragment adopt crossmab light-heavy chain exchange, i.e., VH and VL are exchanged with each other, or the constant regions CL and CH1 are exchanged with each other.

Preferably, the constant regions CL and CH1 in the second antigen-binding fragment are exchanged with each other, the amino acid sequence of the exchanged heavy chain comprising constant region CL is set forth in SEQ ID NO: 76, and the amino acid sequence of the exchanged light chain comprising CH1 is set forth in SEQ ID NO: 77;
and/or, the amino acid sequence of the heavy chain in the first antigen-binding fragment is set forth in SEQ ID NO: 74, and the amino acid sequence of the light chain is set forth in SEQ ID NO: 64.

In one embodiment, the Fc region has reduced effector functions, such as reduced ADCC, ADCP, CDC and/or binding to Clq, FcγRI, FcγRII or FcγRIIIA. For example, the Fc region may be of IgG1 isotype, or non-IgG1 isotype such as IgG2, IgG3 or IgG4, which has been mutated to have reduced or even eliminated ability to mediate effector functions. Such mutations have been described, for example, in Dall'Acqua WF et al., J Immunol. 177(2):1129-1138(2006) and Hezareh M, J Virol.; 75(24):12161-12168(2001). For example, compared to a wild-type sequence, the Fc region may comprise an amino acid sequence having one or more of the following amino acid substitutions: E233P, L234A, L234F, L235A, L235E, G237A, N297A, N297D, P331S and P329G. In a preferred embodiment of the present invention, the Fc region adopts the L234A/L235A/P329G mutation combination.

In some embodiments, the mutated Fc region has enhanced binding affinity to the neonatal Fc receptor (FcRn) compared to a wild-type Fc region; for example, the M252Y/S254T/T256E mutations are introduced into the Fc region.

In some embodiments, the Fc region comprises modification(s) or mutation(s) that can inhibit Fc homodimerization. In some embodiments, the Fc region comprises a variant of the human IgG1 Fc wild-type sequence. Preferably, amino acid residues on one side of the variant Fc domain are replaced with amino acid residues having a larger side chain volume, i.e., a knob mutation, thereby forming a protrusion structure; amino acid residues on the other side are replaced with amino acid residues having a smaller side chain volume, i.e., a hole mutation, thereby forming a cavity structure; and the cavity structure accommodates the protrusion structure so that a heterodimer is formed. Preferably, the mutation scheme of the Fc domain is selected from: (a) knob mutation is T366W, and hole mutations are T366S, L368A and Y407V; (b) knob mutations are S354C and T366W, and hole mutations are Y349C, T366S, L368A and Y407V; wherein the numbering above is according to the EU index.

In some embodiments, in the anti-CD84 bispecific antibodies of the present invention, the second antigen-binding fragment is a scFv comprising the VL2 and the VH2, and the scFv is optionally linked via a linker to the N/C-terminus of VL1 or VH1, or to the N/C-terminus of the Fc region.

The linkers may be those as described above. For example, the linker may be any flexible linker. In some embodiments, the linker comprises an amino acid sequence selected from (G4S)n and GS(G4S)n, wherein n is an integer selected from 1 to 5. Preferably, the linker comprises the amino acid sequence set forth in GGGGS.

In some embodiments, insertions, deletions and/or substitutions may be made in the framework (FR) regions, for example FR1, FR2, FR3 and/or FR4; and/or in the constant regions, for example CL, CH1, CH2 and/or CH3.

In some embodiments, substitution of one or more amino acids may be conservative substitution of one or more amino acids. Examples of conservative substitutions are as described above.

### ii. Polynucleotides, Vectors and Hosts

The present invention provides nucleic acids encoding any of the above anti-CD84 antibodies or fragments thereof. Also provided are vectors comprising the nucleic acids. In one embodiment, the vectors are expression vectors. Also provided are host cells comprising the nucleic acids or the vectors. In one embodiment, the host cells are eukaryotic. In another embodiment, the host cells are selected from yeast cells, mammalian cells (such as CHO cells or 293 cells). In another embodiment, the host cells are prokaryotic.

The present invention also provides polynucleotides encoding at least one CDR region and typically all three CDR regions of the heavy chain VH or light chain VL sequence of antibodies that bind to CD84 as described above. In some further embodiments, the polynucleotides encode the intact or substantially intact variable region sequences of the heavy and/or light chains of the antibodies that bind to CD84 as described above.

As will be appreciated by the skilled in the art, due to codon degeneracy, each antibody or polypeptide amino acid sequence can be encoded by multiple nucleic acid sequences.

In a preferred embodiment, the nucleic acids of the present invention encoding the antibodies also comprise nucleotide sequences encoding the heavy chain constant region.

In one embodiment, one or more vectors comprising the nucleic acids of the present invention are provided. In one embodiment, the vectors are expression vectors, for example, eukaryotic expression vectors. Vectors include, but are not limited to, viruses, plasmids, cosmids, λ phages, or yeast artificial chromosomes (YAC).

In one embodiment, host cells comprising the vectors are provided. Suitable host cells for cloning or expressing antibody-encoding vectors include prokaryotic or eukaryotic cells described herein. For example, antibodies may be produced in bacteria, particularly when glycosylation and Fc effector functions are not required. For expression of antibody fragments and polypeptides in bacteria, see, e.g., U.S. Patent Nos. 5,648,237, 5,789,199, and 5,840,523; see also Charlton, Methods in Molecular Biology, Vol. 248 (B.K.C. Lo, ed., Humana Press, Totowa, NJ, 2003), pp. 245-254, which describes expression of antibody fragments in *E. coli*. After expression, antibodies may be isolated from bacterial cell pastes in soluble fractions and can be further purified.

In one embodiment, the host cell is eukaryotic. In another embodiment, the host cell is selected from yeast cells, mammalian cells, or other cells suitable for preparing antibodies or antigen-binding fragments thereof. For example, eukaryotic microorganisms such as filamentous fungi or yeast are suitable cloning or expression hosts for vectors encoding antibodies. For example, fungal and yeast strains whose glycosylation pathways have been "humanized" result in the production of antibodies with partially or fully human glycosylation patterns. See Gerngross, Nat. Biotech. 22: 1409-1414(2004), and Li et al., Nat. Biotech. 24: 210-215(2006). Host cells suitable for expressing glycosylated antibodies are also derived from multicellular organisms (invertebrates and vertebrates). Vertebrate cells may also be used as hosts. For example, mammalian cell lines modified to be suitable for suspension growth may be used. Other examples of useful mammalian host cell lines are monkey kidney CV1 line transformed with SV40 (COS-7); human embryonic kidney line (293 HEK or 293 cells, as described, e.g., in Graham et al., J. Gen Virol. 36: 59(1977)), etc. Other useful mammalian host cell lines include Chinese hamster ovary (CHO) cells, including DHFR-CHO cells (Urlaub et al., Proc. Natl. Acad. Sci. USA 77: 216(1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268(2003).

### iii. Chimeric Antigen Receptors

In one aspect of the present invention, the present invention relates to an isolated chimeric antigen receptor (CAR) molecule comprising (e.g., sequentially linked) an extracellular binding region that binds to CD84, a transmembrane region, and an intracellular signaling region (e.g., comprising a costimulatory domain).

In one example of the present invention, the transmembrane region particularly used in the present invention may include at least the following transmembrane regions: for example, CD28, CD8 (e.g., CD8α, CD8β). In some cases, the transmembrane region may be linked via a hinge (e.g., a hinge from a human protein) to the extracellular region of the CAR, e.g., the antigen-binding domain of the CAR. For example, in one embodiment, the hinge may be a human Ig (immunoglobulin) hinge (e.g., IgG4 hinge, IgD hinge), GS linker, KIR2DS2 hinge, or CD8 hinge.

In one example of the present invention, the intracellular domain of the CAR comprises a CD3ζ chain, which provides a T cell receptor activation signal and proliferation stimulation for genetically modified T cells. In addition to comprising the TCRξ chain, the intracellular domain also comprises one CD28 or 4-1BB costimulatory signaling domain. The CD28 domain enhances the initial activation and proliferation of CAR-T cells and improves effector functions, while the 4-1BB domain mainly improves the expansion and long-term viability of CAR-T cells.

In addition, the intracellular domain co-expresses some small molecule substances (such as the pro-inflammatory cytokine IL-12), which can trigger cytokine-induced signals or block some signaling pathways that affect the function of CAR-T cells.

### iv. Immunoconjugates

In a further aspect, the present invention provides immunoconjugates produced by conjugating antibodies of the present invention or antigen-binding fragments thereof to heterologous molecules. In one embodiment, in the immunoconjugates, the antibodies of the present invention or the antigen-binding fragments thereof are conjugated to therapeutic agents or diagnostic agents. In some embodiments, the antibodies of the present invention may be conjugated to heterologous molecules in the form of a full-length antibody or an antibody fragment. For example, conjugation is performed in the fragment form of a Fab fragment, a Fab' fragment, a F(ab)'2 fragment, a single-chain scFab antibody, a single-chain scFv, a VHH, etc.

Linkers may be used to covalently link different entities of the conjugate. Suitable linkers include chemical linkers or peptide linkers. Advantageously, the linker is a "cleavable linker" that facilitates release of a polypeptide after delivery to a target site. For example, acid-labile linkers, peptidase-sensitive linkers, photolabile linkers, dimethyl linkers, or disulfide-containing linkers may be used.

Therapeutic agents suitable for conjugates include, but are not limited to, cytotoxins (e.g., cytostatic or cytocidal agents), drugs, or radioisotopes. Examples of cytotoxic agents (e.g., chemotherapeutic agents) suitable for forming immunoconjugates are known in the art, see, e.g., WO 05/103081. For example, cytotoxic agents include, but are not limited to: radioisotopes; growth inhibitors; enzymes and fragments thereof such as nucleases; antibiotics; toxins such as small molecule toxins or enzymatically active toxins of bacterial, fungal, plant or animal origin, including fragments and/or variants thereof; and various known anti-tumor or anti-cancer agents.

In a further aspect, the antibodies of the present invention may be conjugated to diagnostic or detectable agents. Such conjugates may be used to monitor or predict the onset, formation, progression and/or severity of a disease or condition as part of a clinical testing method (e.g., determining the efficacy of a particular therapy). Such diagnosis and detection can be achieved by conjugating the antibodies to a detectable agents including, but not limited to, various enzymes such as, but not limited to, horseradish peroxidase; prosthetic groups such as, but not limited to, streptavidin/biotin and avidin/biotin; fluorescent materials; luminescent materials; radioactive materials; and positron-emitting metals and non-radioactive paramagnetic metal ions used in various positron emission imaging techniques.

### v. Pharmaceutical Compositions and Pharmaceutical Formulations

The present invention also includes compositions (including pharmaceutical compositions or pharmaceutical formulations) comprising anti-CD84 antibodies or immunoconjugates thereof or chimeric antigen receptors thereof, and compositions comprising polynucleotides encoding anti-CD84 antibodies or immunoconjugates thereof or chimeric antigen receptors thereof. These compositions may also optionally comprise suitable pharmaceutical excipients, such as pharmaceutical carriers and pharmaceutical vehicles known in the art, including buffers.

Pharmaceutical carriers suitable for the present invention may be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions may also be used as liquid carriers, particularly for injectable solutions. Suitable pharmaceutical vehicles include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. These compositions may take the form of solutions, suspensions, emulsions, tablets, pills, capsules, powders, sustained-release formulations and the like. Oral formulations may contain standard carriers such as pharmaceutical-grade mannitol, lactose, starch, magnesium stearate, saccharin.

Pharmaceutical formulations of the present invention can be prepared by mixing anti-CD84 antibodies, immunoconjugates, or chimeric antigen receptors of the present invention having the desired purity with one or more optional pharmaceutical excipients (Remington's Pharmaceutical Sciences, 16th edition, Osol, A., ed. (1980)), preferably in the form of lyophilized formulations or aqueous solutions.

In the pharmaceutical compositions and pharmaceutical formulations of the present invention, the antibodies of the present invention may be the sole active agent or may be combined with other therapeutic agents. Therapeutic agents that can be combined with the antibodies of the present invention include, but are not limited to, those having beneficial therapeutic efficacy for the disease and/or condition to be treated. For example, the active ingredients may be required for the particular indication being treated, preferably those active ingredients having complementary activities that do not adversely affect each other. For example, other pharmaceutical ingredients that can provide anti-cancer activity. The antibodies of the present invention and the active ingredients present in the pharmaceutical compositions and pharmaceutical formulations are suitably combined in amounts effective for the intended use.

Sustained-release formulations can be prepared. Suitable examples of sustained-release formulations include semipermeable matrices of solid hydrophobic polymers comprising the antibodies, and the matrices are in the form of shaped articles, e.g., films or microcapsules.

### vi. Combination Products

In a further aspect, the present invention also provides a combination product comprising antibodies or antigen-binding fragments thereof, immunoconjugates or chimeric antigen receptors of the present invention, and one or more other therapeutic agents (e.g., chemotherapeutic agents, other antibodies, cytotoxic agents, anti-tumor drugs, etc.). The combination product of the present invention can be used in the treatment methods of the present invention. In some embodiments, the present invention provides a combination product, wherein the other therapeutic agents are, for example, a therapeutic agent such as an antibody that effectively stimulates an immune response to further enhance, stimulate or upregulate the immune response of a subject.

In some embodiments, the combination product is used for the prevention or treatment of a tumor. In some embodiments, the tumor is a hematological malignancy, such as acute myeloid leukemia (AML), chronic lymphocytic leukemia (CLL), blastic plasmacytoid dendritic cell neoplasm (BPDCN), chronic myeloid leukemia (CML), myelodysplastic syndrome (MDS), acute lymphoblastic leukemia (ALL), multiple myeloma (MM), hairy cell leukemia (HCL), or Hodgkin's lymphoma (HL).

### vii. Therapeutic/Preventive/Diagnostic Uses

In another aspect, the present invention also provides a method of treating and/or preventing and/or diagnosing a disease associated with CD84 expression, comprising administering to a subject the humanized antibodies, chimeric antigen receptors, immunoconjugates, or pharmaceutical compositions as described above.

In one embodiment, the disease associated with CD84 expression includes, but is not limited to, CD84-positive acute myeloid leukemia (AML), chronic lymphocytic leukemia (CLL), blastic plasmacytoid dendritic cell neoplasm (BPDCN), chronic myeloid leukemia (CML), myelodysplastic syndrome (MDS), acute lymphoblastic leukemia (ALL), multiple myeloma (MM), hairy cell leukemia (HCL), or Hodgkin's lymphoma (HL).

### viii. Detection Applications

The present invention also provides methods and kits for detecting CD84 in a sample, wherein the method comprises: (a) contacting the sample with the antibodies of the present invention or antigen-binding fragments or immunoconjugates thereof; and (b) detecting the formation of a complex between the antibodies, or antigen-binding fragments or immunoconjugates or chimeric antigen receptors thereof, and CD84 proteins. In some embodiments, the sample is from a cancer patient, such as a leukemia patient. The detection may be *in vitro* or *in vivo*.

As used herein, the term "detection" includes quantitative or qualitative detection, and exemplary detection methods may involve immunohistochemistry, immunocytochemistry, flow cytometry (e.g., FACS), magnetic beads complexed with antibody molecules, ELISA assays, PCR techniques (e.g., RT-PCR). In certain embodiments, the biological sample is blood, serum, or other liquid samples of biological origin. In certain embodiments, the biological sample comprises cells or tissues. In some embodiments, the biological sample is from a hyperproliferative or cancerous lesion. In certain embodiments, the CD84 to be detected is human CD84.

In one embodiment, anti-CD84 antibodies are used to select a subject suitable for treatment utilizing anti-CD84 antibodies, for example wherein CD84 is a biomarker for selecting the subject. In one embodiment, the antibodies of the present invention can be used to diagnose cancer or tumor, for example to assess (e.g., monitor) the treatment or progression, diagnosis and/or staging of a disease described herein (e.g., a hyperplastic or cancerous disease) in a subject.

In certain embodiments, labeled anti-CD84 antibodies are provided. Labels include, but are not limited to, labels or portions that are directly detected (such as fluorescent labels, chromophore labels, electron-dense labels, chemiluminescent labels, and radioactive labels), and portions that are indirectly detected, such as enzymes or ligands, for example, via enzymatic reactions or molecular interactions. Exemplary labels include, but are not limited to, radioactive isotopes 32P, 14C, 125I, 3H, and 131I; fluorophores such as rare earth chelates or fluorescein and derivatives thereof; rhodamine and derivatives thereof; dansyl; umbelliferone; luciferase, e.g., firefly luciferase and bacterial luciferase (U.S. Patent No. 4,737,456); luciferin; 2,3-dihydrophthalazinedione; horseradish peroxidase (HR); alkaline phosphatase; β-galactosidase; glucoamylase; lyase; carbohydrate oxidases such as glucose oxidase, galactose oxidase, and glucose-6-phosphate dehydrogenase; heterocyclic oxidases such as uricase and xanthine oxidase; as well as enzymes that use hydrogen peroxide to oxidize a dye precursor such as HR, lactoperoxidase, or microperoxidase; biotin/avidin; spin labels; phage labels; stable free radicals; and the like.

The following examples are described to assist in understanding the present invention. The examples are not intended and should not be construed as limiting the protection scope of the present invention in any way.

### EMBODIMENTS OF THE INVENTION

### Example 1 Construction of CD84 Overexpressing Cell Lines

### 1.1 Construction Methods of HEK293T-hCD84 and HEK293T-Cyno-CD84 Overexpressing Cells

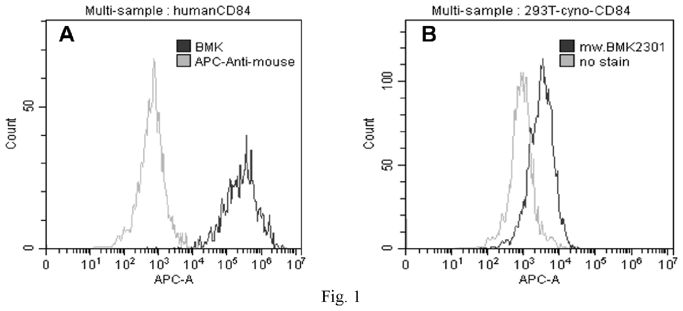

Human CD84 and cynomolgus CD84 sequences were both obtained from the NCBI database with Accession Nos. NP_001171808.1 and XM_005541244.3, respectively. First, CD84 sequences of different species were individually cloned into the lentiviral overexpression vector pCDH-CMV.MCS-EF1.CopGFP.T2A.Puro by PCR technology. The constructed overexpression plasmids and viral packaging plasmids (psPAX2 and pMD2.G) were then transfected into HEK293T cells in a certain proportion via lipofectae 2000 for viral packaging. Supernatants containing the virus were collected approximately 48 h later. HEK293T cells were infected with the collected viral supernatants to construct HEK293T-human-CD84 and HEK293T-cyno-CD84 cell lines. All overexpressing cell lines were cultured using DMEM + 10% FBS + 1% P/S + 5 µg/mL puromycin during expansion. The constructed overexpressing cell lines were evaluated using CD84.1.21 (Biolegend; Cat. No. 326002) as the positive control molecule (BMK). The construction results are shown in Fig. 1: both HEK293T-hCD84 and HEK293T-Cyno-CD84 overexpressing cell lines were successfully constructed.

### Example 2: Screening and Preparation of Hybridoma Cells and Monoclonal Antibodies

### 2.1 Preparation, Screening and Identification of Hybridoma Cells

Spleens were harvested from immunized mice under sterile conditions. The spleens were gently crushed, then ground, and passed through a 40-mesh sieve, and cells were separated into sterile PBS. The cells were washed twice by centrifugation at 1500 rpm for 5 min repeatedly, and then resuspended using a certain volume of electrotransfer buffer, allowing them to stand for 5 min. Mouse myeloma cells were collected in PBS, and myeloma cells (SP20) were washed twice by centrifugation at 1500 rpm for 5 min in the same manner as that for the splenocytes treatment, and then resuspended using a certain volume of electrotransfer buffer, allowing them to stand for 5 min. The mouse splenocytes and myeloma cells were then thoroughly mixed at a ratio of 1.5:1. Electrofusion was performed on an electroporator under preset conditions. After fusion, an appropriate amount of medium (DMEM + 10% FBS + 1% HT + 1% P/S) was added thereto and the cells were transferred to a 96-well flat-bottom plate at an amount of 2E4 cells/well. After approximately 7-10 days, when the cells grew to a certain quantity, the hybridoma supernatants were identified according to a preset screening protocol to obtain clones producing CD84-specific antibodies. For a hybridoma screening, human and cynomolgus CD84 proteins were firstly used to screen the hybridoma supernatants by employing ELISA. Then the ELISA-positive samples were further subjected to binding and cross-species detection by flow cytometry. Finally, the ELISA- and FCM-positive clones were selected for verification by ligand blocking assay to determine the final positive clonal molecules.

### 2.2 Preparation of Chimeric Antibody Molecules and Determination of Binding Activity to 239T-hCD84 Cells

### Expression of Chimeric Antibodies

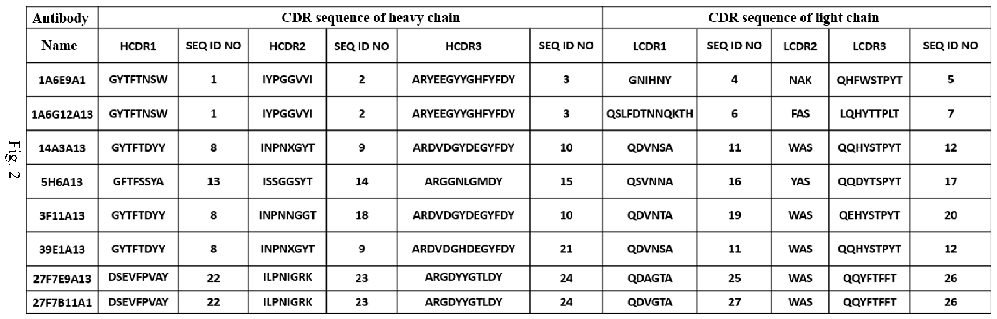

Heavy chain sequences of the positive clone molecules obtained by the above screening were entrusted to a service organization for routine synthesis, and were respectively cloned into the pTT5-hIgG1.CH vector containing the IgG1 heavy chain constant region amino acid sequence by homologous recombination to obtain chimeric antibody heavy chain expression plasmids. Light chain sequences of the positive clone molecules obtained by the above screening were entrusted to a service organization for routine synthesis, and were respectively cloned into the pTT5-hKappa.CL vector by homologous recombination. 293F cells in the logarithmic growth phase with good growth status were seeded into a 250 mL cell culture flask and cultured in 50 mL of medium, and 25 µg of the light and heavy chain expression plasmids were each co-transfected with PEI. Cell supernatants were collected on day 7 of the post-transfection culture, centrifuged, and filtered using a 0.45 µm filter. Antibodies were purified using Protein A medium and exchanged into PBS buffer of pH 7.2 by dialysis. Antibody concentration and purity were determined by measuring absorbance with Nanodrop, and purity was examined by sodium dodecyl sulfate gel electrophoresis and Coomassie staining. Finally, 8 chimeric antibodies were obtained (**1A6E9A1, 1A6G12A13, 14A3A13, 5H6A13, 3F11A13, 39E1A13, 27F7E9A13, 27F7B11A1**). The amino acid sequences of the CDR regions of the above chimeric antibodies are listed in Fig. 2.

### Flow Cytometry Screening

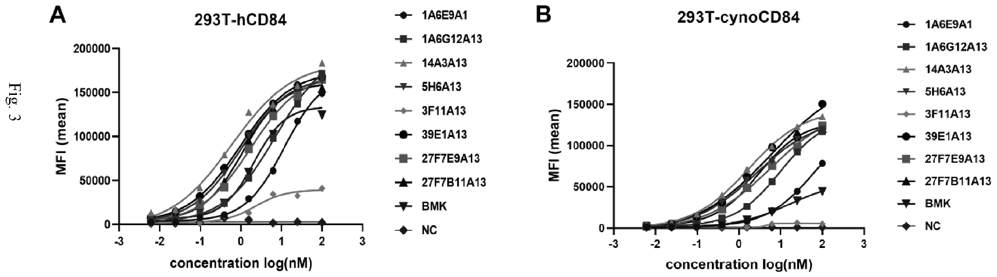

Pre-cultured cells were trypsinized and then resuspended in the FACS Buffer (PBS + 1% BSA) solution. After cell counting, cells were seeded into a 96-well V-bottom plate at the amount of 2E5 cells/100 µL per well. After the cell treatment was completed, antibodies were added to each well in a volume of 100 µL at a preset concentration, mixed well with the cells, and then incubated at 4°C for 1 h, which were centrifuged at 1500 rpm/min for 5 min after the incubation. The supernatant was discarded and the cell pellet was retained, and the cells were resuspended by adding 200 µL of FACS Buffer. Centrifugation and washing were repeated twice under the same conditions. Then, 100 µL of the secondary antibody was added thereto and incubation was continued for 0.5 h, followed by centrifugation and washing twice. Finally, the cells were resuspended by adding 100 µL of FACS Buffer and subjected to detection on an instrument. The number of cells per sample should not be less than 10,000 during detection. The experimental results are shown in Table 1 and Fig. 3. Compared with the control antibody BMK, the chimeric antibodies 1A6G12A13, 14A3A13, 5H6A13, and 39E1A13 exhibited superior binding ability to HEK293T-hCD84 and HEK293T-Cyno-CD84 cells.

**Table 1 Affinity Assay Results of Chimeric Antibodies**

| | **HEK293T-hCD84** | | **HEK293T-Cyno-CD84** | |
|---|---|---|---|---|
| **Antibody Name** | **EC50 (nM)** | **MFI (max)** | **EC50 (nM)** | **MFI (max)** |
| **1A6E9A1** | 11.780 | 171244 | 65.860 | 135636 |
| **1A6G12A13** | 7.217 | 198882 | 9.831 | 135468 |
| **14A3A13** | 0.591 | 184437 | 1.704 | 144210 |
| **5H6A13** | 0.993 | 168393 | 2.564 | 133263 |
| **3F11A13** | 2.280 | 39244 | 4.749 | 5898 |
| **39E1A13** | 0.943 | 174922 | 5.975 | 185858 |
| **27F7E9A13** | 1.759 | 171860 | 5.900 | 148772 |
| **27F7B11A1** | 1.131 | 161267 | 2.988 | 129774 |
| **BMK** | 2.088 | 134190 | 16.590 | 57683 |

### Example 3 Humanization of the Anti-CD84 Antibodies of the Present Invention

The obtained chimeric antibodies 1A6G12A13 (abbreviated as 1A6 in the following examples), 14A313 (abbreviated as 14A3), 39E1A13 (abbreviated as 39E1), and 5H6A13 (abbreviated as 5H6) were humanized. The steps were as follows:
(1) CDR sequences were determined according to the extended Kabat and IMGT CDR numbering schemes;
(2) The closest homologous sequences were found for each V/J region of the heavy and light chains in the human germline sequence database;
(3) Human germlines with the best matching to the heavy and light chains and with the minimum amount of back-mutations were screened;
(4) The CDR regions of the chimeric antibodies were grafted onto the human framework regions;
(5) Amino acid positions in the framework regions that maintain the CDR function were identified using sequence and structural characteristics;
(6) Back-mutations were performed at the identified important sequence positions;
(7) Amino acids at risk sites were optimized.

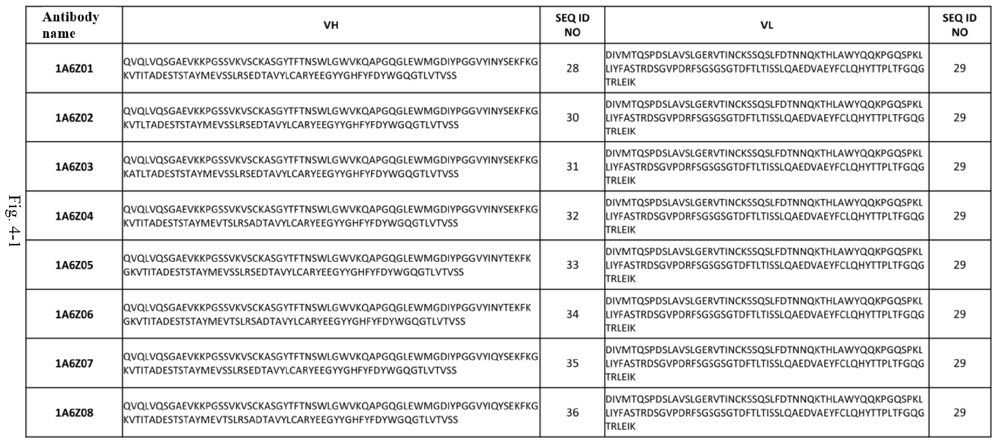

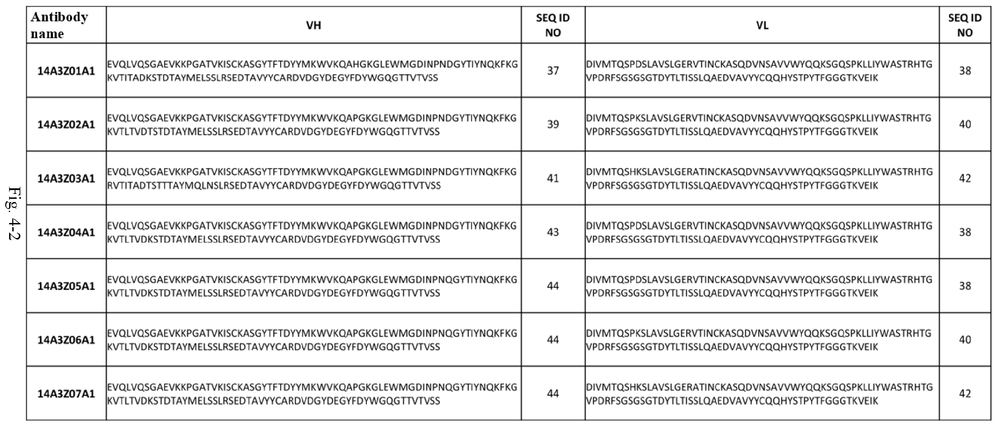

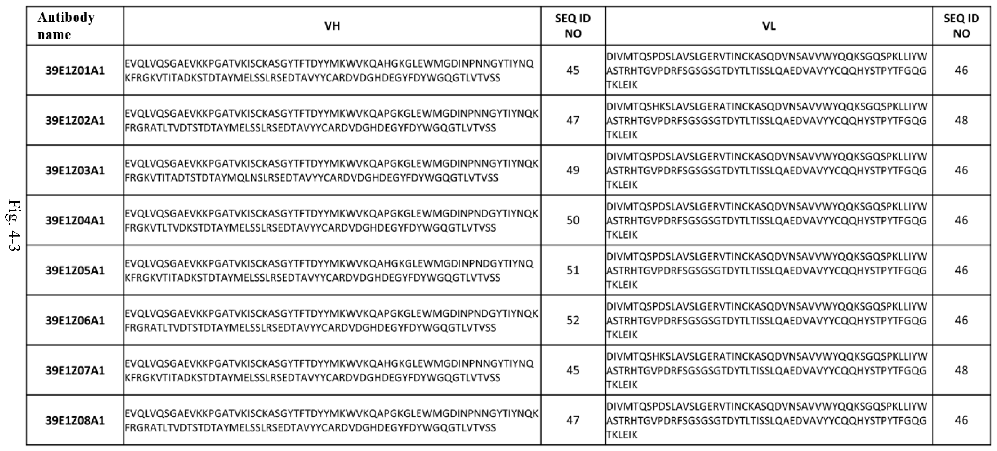

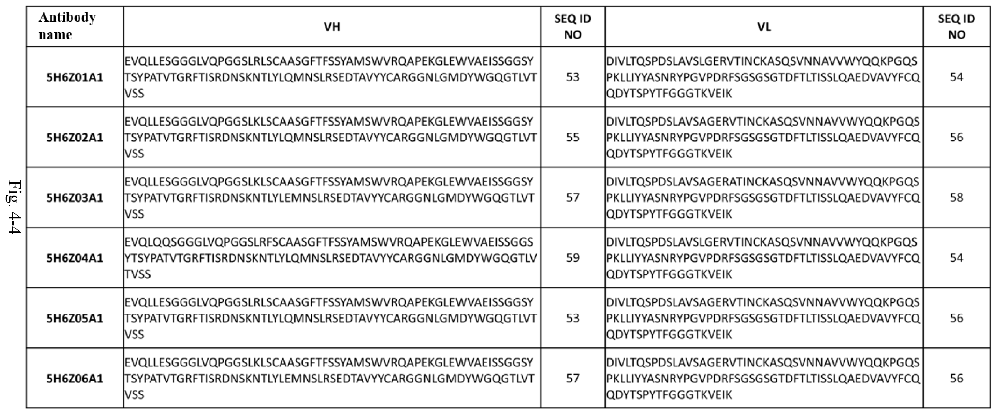

The light chain variable regions and heavy chain variable regions of the obtained humanized antibodies are shown in Fig. 4 for specific sequence information.

### Example 4 Purification and Expression of the Anti-CD84 Humanized Antibodies of the Present Invention

293F cells were used to transiently express the CD84 humanized antibodies constructed in Example 3, and the supernatants were collected and purified with Protein A to obtain the antibodies. The humanized variable region sequences of the heavy and light chains were synthesized by BioSune Biotech (Shanghai) Co., Ltd. and cloned into the pTT5-hIgG1.CH vector containing the IgG1 heavy chain constant region amino acid sequence and the pTT5-hKappa.CL vector, respectively, to obtain antibody expression plasmids. 293F cells in the logarithmic growth phase with good growth status were seeded into a 250 mL cell culture flask and cultured in 50 mL of medium. The vectors carrying the heavy and light chains of the antibody molecules were transfected into 293F cells using chemical transfection. The chemical transfection reagent used was PEI (purchased from Polysciences), and the cultured 293F cells were transiently transfected according to the protocol provided by the manufacturer. Cell supernatants were collected on day 5 of the post-transfection culture, centrifuged at 4000 rpm for 20 min, and the supernatants were taken and purified using Protein A matrix (purchased from Bestchrom). The antibodies were then exchanged into PBS buffer (pH 7.2) by dialysis.

### Example 5 Detection of Binding of Humanized Antibodies to Overexpressing Cells

### 5.1 Affinity Determination of Humanized Antibodies to 293T-hCD84, HEL and U937 Cells

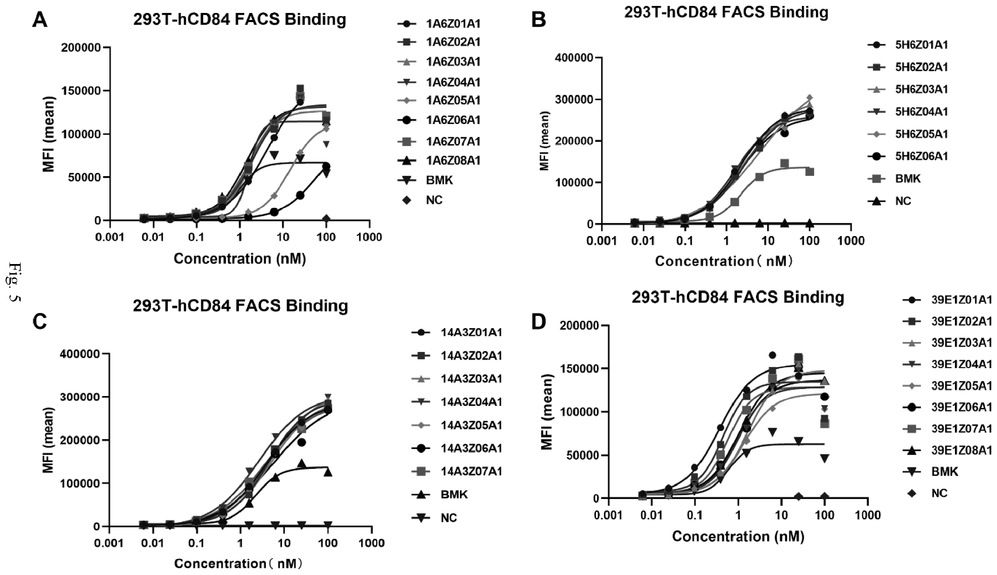

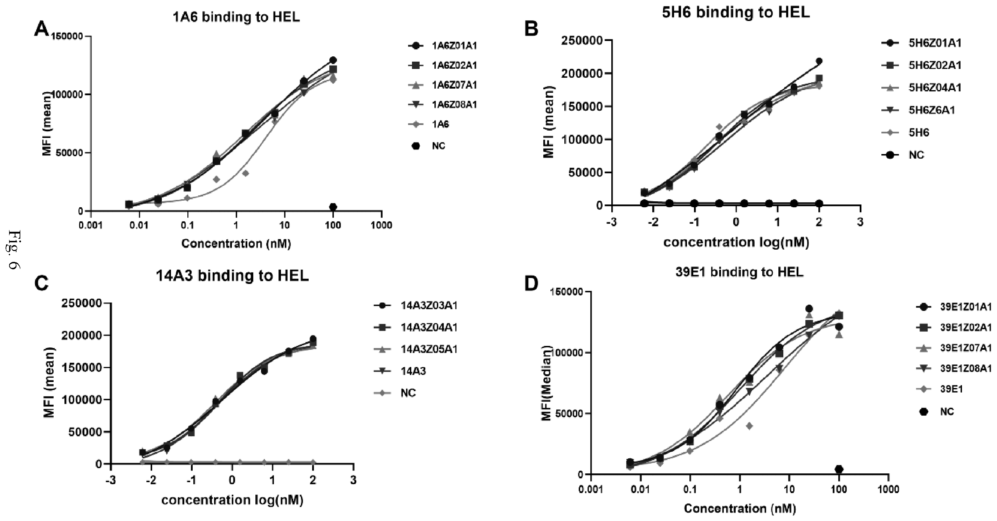

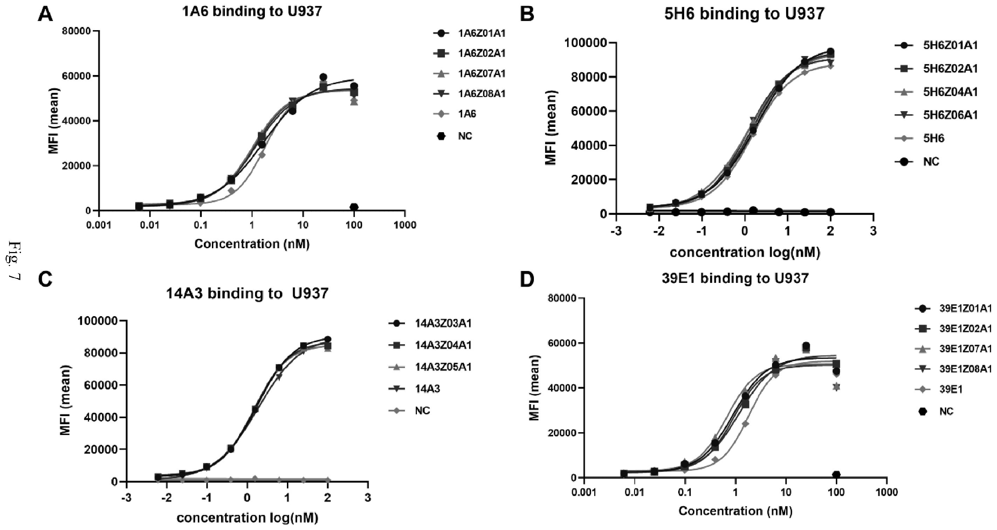

Pre-cultured cells were rypsinized and then resuspended in the FACS Buffer (PBS + 1% BSA) solution. After cell counting, cells were seeded into a 96-well V-bottom plate at the amount of 2E5 cells/100 µL per well. After the cell treatment was completed, antibodies were added to each well in a volume of 100 µL at a preset concentration, mixed well with the cells, and then incubated at 4°C for 1 h, which were centrifuged at 1500 rpm/min for 5 min after the incubation. The supernatant was discarded, and the cell pellet was retained, and the cells were resuspended by adding 200 µL of FACS buffer. Centrifugation and washing were repeated twice under the same conditions. Then, 100 µL of the secondary antibody was added thereto, and incubation was continued for 0.5 h, followed by centrifugation and washing twice. Finally, the cells were resuspended by adding 100 µL of FACS Buffer and subjected to detection on an instrument. The number of cells per sample should not be less than 10,000 during detection. The experimental results are shown in Figs. 5, 6 and 7. The humanized antibody molecules 5H6Z01A1-5H6Z06A1 derived from the chimeric antibody molecule 5H6A13 all showed strong binding ability to human CD84 at cellular level. The humanized antibody molecules 14A3Z01A1-14A3Z07A1 derived from the chimeric antibody molecule 14A3A13 all showed strong binding ability to human CD84 at cellular level. Except for 1A6Z05A1 and 1A6Z06A1, the other humanized antibody molecules derived from the chimeric antibody molecule 1A6E9A1 showed strong binding ability to human CD84 at cellular level. The humanized antibody molecules 39E1Z01A1-39E1Z08A1 derived from the chimeric antibody molecule 39E1A13 all showed strong binding ability to human CD84 at cellular level.

### Example 6 Preparation of CD84×CD3 Bispecific Antibodies

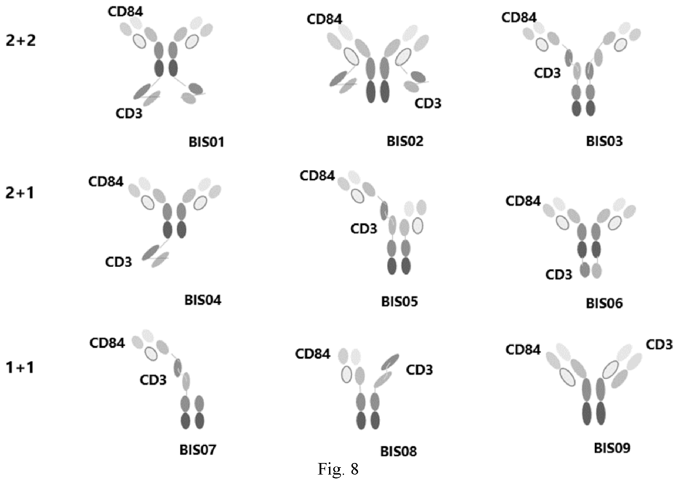

According to the combinations in Table 2, anti-CD84 clone 39E1Z01A1 and anti-CD3 clone (VH: SEQ ID NO: 61; VL: SEQ ID NO: 62) were combined to construct a total of 9 bispecific antibodies, the structures of which are shown in Fig. 8. The peptide chains corresponding to the above bispecific antibodies were respectively cloned into vectors to obtain antibody expression plasmids. 293F cells in the logarithmic growth phase with good growth status were seeded into a 250 mL cell culture flask and cultured in 50 mL of medium. The vectors carrying the heavy and light chains of the antibody molecules were transfected into 293F cells using chemical transfection. The chemical transfection reagent used was PEI (purchased from Polysciences), and the cultured 293F cells were transiently transfected according to the protocol provided by the manufacturer. Cell supernatants were collected on day 5 of the post-transfection culture, centrifuged at 4000 rpm for 20 min, and the supernatants were taken and purified using Protein A matrix (purchased from Bestchrom). The antibodies were then exchanged into PBS buffer of pH 7.2 by dialysis, and further purified to obtain the corresponding bispecific antibodies.

**Table 2 Sequence Information of CD84×CD3 Bispecific Antibodies**

| **Bispecific Antibody Sequence Information** | |
|---|---|
| BIS01 | |
| SEQ ID NO:63 | Chain 1: VH-CH-Fc (L234A/L235A/M252Y/S254T/T256E/P329G)-Linker-Scfv |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGKGGGGSE VQLVESGGGLVQPGGSLRLSCAASGFTFNTYAMNWVRQAPGKGLEWVARIRSKYNN YATYYAASVKGRFTISRDDSKNSLYLQMNSLKTEDTAVYYCARHGNFGDSYVSWFAY WGQGTLVTVSSGGGGSGGGGSGGGGSQTVVTQEPSLTVSPGGTVTLTCRSSTGAVTT SNYANWVQQKPGQAPRGLIGGTNKRAPGTPARFSGSLLGGKAALTLSGVQPEDEAEY YCALWYSNHWVFGGGTKLTVL | |
| SEQ ID NO:64 | Chain 2: VL-CL (CD84) |
| DIVMTQSPDSLAVSLGERVTINCKASQDVNSAVVWYQQKSGQSPKLLIYWASTRHTG VPDRFSGSGSGTDYTLTISSLQAEDVAVYYCQQHYSTPYTFGQGTKLEIKRTVAAPSVF IFPPSDEQLKSGTASVVCLLNNFYPREAKVQWKVDNALQSGNSQESVTEQDSKDSTY SLSSTLTLSKADYEKHKVYACEVTHQGLSSPVTKSFNRGEC | |
| BIS02 | |
| SEQ ID NO:65 | Chain 1: VH-CH-Fc(L234A/L235A/M252Y/S254T/T256E/P329G) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK | |
| SEQ ID NO:66 | Chain 2: VL-CL-linker-Scfv |
| DIVMTQSPDSLAVSLGERVTINCKASQDVNSAVVWYQQKSGQSPKLLIYWASTRHTG VPDRFSGSGSGTDYTLTISSLQAEDVAVYYCQQHYSTPYTFGQGTKLEIKRTVAAPSVF IFPPSDEQLKSGTASVVCLLNNFYPREAKVQWKVDNALQSGNSQESVTEQDSKDSTY SLSSTLTLSKADYEKHKVYACEVTHQGLSSPVTKSFNRGECGGGGSEVQLVESGGGL VQPGGSLRLSCAASGFTFNTYAMNWVRQAPGKGLEWVARIRSKYNNYATYYAASVK GRFTISRDDSKNSLYLQMNSLKTEDTAVYYCARHGNFGDSYVSWFAYWGQGTLVTVS SGGGGSGGGGSGGGGSQTVVTQEPSLTVSPGGTVTLTCRSSTGAVTTSNYANWVQQK PGQAPRGLIGGTNKRAPGTPARFSGSLLGGKAALTLSGVQPEDEAEYYCALWYSNHW VFGGGTKLTVL | |
| BIS03 | |
| SEQ ID NO:67 | Chain 1: VH-CH-Linker-Scfv-Fc (L234A/L235A/M252Y/S254T/T256E/P329G) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCGGG GSEVQLVESGGGLVQPGGSLRLSCAASGFTFNTYAMNWVRQAPGKGLEWVARIRSK YNNYATYYAASVKGRFTISRDDSKNSLYLQMNSLKTEDTAVYYCARHGNFGDSYVS WFAYWGQGTLVTVSSGGGGSGGGGSGGGGSQTVVTQEPSLTVSPGGTVTLTCRSSTG AVTTSNYANWVQQKPGQAPRGLIGGTNKRAPGTPARFSGSLLGGKAALTLSGVQPED EAEYYCALWYSNHWVFGGGTKLTVLDKTHTCPPCPAPEAAGGPSVFLFPPKPKDTLYI TREPEVTCVVVDVSHEDPEVKFNWYVDGVEVHNAKTKPREEQYNSTYRVVSVLTVL HQDWLNGKEYKCKVSNKALGAPIEKTISKAKGQPREPQVYTLPPSREEMTKNQVSLT CLVKGFYPSDIAVEWESNGQPENNYKTTPPVLDSDGSFFLYSKLTVDKSRWQQGNVFS CSVMHEALHNHYTQKSLSLSPGK | |
| SEQ ID NO:64 | Chain 2: VL-CL (CD84) |
| BIS04 | |
| SEQ ID NO:68 | Chain 1: VH-CH-Fc (L234A/L235A/M252Y/S254T/T256E/P329G/S354C/T366W)-Linker-Scfv (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGKGGGGS EVQLVESGGGLVQPGGSLRLSCAASGFTFNTYAMNWVRQAPGKGLEWVARIRSKYN NYATYYAASVKGRFTISRDDSKNSLYLQMNSLKTEDTAVYYCARHGNFGDSYVSWFA YWGQGTLVTVSSGGGGSGGGGSGGGGSQTVVTQEPSLTVSPGGTVTLTCRSSTGAVT TSNYANWVQQKPGQAPRGLIGGTNKRAPGTPARFSGSLLGGKAALTLSGVQPEDEAE YYCALWYSNHWVFGGGTKLTVL | |
| SEQ ID NO:69 | Chain 2: VH-CH-Fc (L234A/L235A/M252Y/S254T/T256E/P329G/Y349C/T366S/L368A/Y407V) (Hole) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVCTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGK | |
| SEQ ID NO:64 | Chain 3: VL-CL (CD84) |
| DIVMTQSPDSLAVSLGERVTINCKASQDVNSAVVWYQQKSGQSPKLLIYWASTRHTG VPDRFSGSGSGTDYTLTISSLQAEDVAVYYCQQHYSTPYTFGQGTKLEIKASVAAPSVF IFPPSDEQLKSGTASVVCLLNNFYPREAKVQWKVDNALQSGNSQESVTEQDSKDSTY SLSSTLTLSKADYEKHKVYACEVTHQGLSSPVTKSFNRGEC | |
| BIS05 | |
| SEQ ID NO:70 | Chain 1: VH-CH-Linker-Scfv-Fc (L234A/L235A/M252Y/S254T/T256E/P329G/S354C/T366W) (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCGGG GSEVQLVESGGGLVQPGGSLRLSCAASGFTFNTYAMNWVRQAPGKGLEWVARIRSK YNNYATYYAASVKGRFTISRDDSKNSLYLQMNSLKTEDTAVYYCARHGNFGDSYVS WFAYWGQGTLVTVSSGGGGSGGGGSGGGGSQTVVTQEPSLTVSPGGTVTLTCRSSTG AVTTSNYANWVQQKPGQAPRGLIGGTNKRAPGTPARFSGSLLGGKAALTLSGVQPED EAEYYCALWYSNHWVFGGGTKLTVLDKTHTCPPCPAPEAAGGPSVFLFPPKPKDTLYI TREPEVTCVVVDVSHEDPEVKFNWYVDGVEVHNAKTKPREEQYNSTYRVVSVLTVL | |
| HQDWLNGKEYKCKVSNKALGAPIEKTISKAKGQPREPQVYTLPPCREEMTKNQVSL WCLVKGFYPSDIAVEWESNGQPENNYKTTPPVLDSDGSFFLYSKLTVDKSRWQQGNV FSCSVMHEALHNHYTQKSLSLSPGK | |
| SEQ ID NO:69 | Chain 2: VH-CH-Fc (L234A/L235A/M252Y/S254T/T256E/P329G/Y349C/T366S/L368A/Y407V) (Hole) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVCTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGK | |
| SEQ ID NO:64 | Chain 3: VL-CL (CD84) |
| BIS06 | |
| SEQ ID NO:71 | Chain 1: VH-CH-Fc (L234A/L235A/M252Y/S254T/T256E/P329G/S354C/T366W)-Linker-VH (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGKGGGGS EVQLVESGGGLVQPGGSLRLSCAASGFTFNTYAMNWVRQAPGKGLEWVARIRSKYN NYATYYAASVKGRFTISRDDSKNSLYLQMNSLKTEDTAVYYCARHGNFGDSYVSWFA YWGQGTLVTVSS | |
| SEQ ID NO:72 | Chain 2: VH-CH-Fc (L234A/L235A/M252Y/S254T/T256E/P329G/Y349C/T366S/L368A/Y407V)-Linker-VL (Hole) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVCTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGKGGGGS DIVMTQSPDSLAVSLGERVTINCKASQDVNSAVVWYQQKSGQSPKLLIYWASTRHTG VPDRFSGSGSGTDYTLTISSLQAEDVAVYYCQQHYSTPYTFGQGTKLEIK | |
| SEQ ID | Chain 3: VL-CL (CD84) |
| NO:64 | |
| BIS07 | |
| SEQ ID NO:70 | Chain 1: VH-CH-Linker-Scfv-Fc (L234A/L235A/M252Y/S254T/T256E/P329G/S354C/T366W) (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCGGG GSEVQLVESGGGLVQPGGSLRLSCAASGFTFNTYAMNWVRQAPGKGLEWVARIRSK YNNYATYYAASVKGRFTISRDDSKNSLYLQMNSLKTEDTAVYYCARHGNFGDSYVS WFAYWGQGTLVTVSSGGGGSGGGGSGGGGSQTVVTQEPSLTVSPGGTVTLTCRSSTG AVTTSNYANWVQQKPGQAPRGLIGGTNKRAPGTPARFSGSLLGGKAALTLSGVQPED EAEYYCALWYSNHWVFGGGTKLTVLDKTHTCPPCPAPEAAGGPSVFLFPPKPKDTLYI TREPEVTCVVVDVSHEDPEVKFNWYVDGVEVHNAKTKPREEQYNSTYRVVSVLTVL HQDWLNGKEYKCKVSNKALGAPIEKTISKAKGQPREPQVYTLPPCREEMTKNQVSL WCLVKGFYPSDIAVEWESNGQPENNYKTTPPVLDSDGSFFLYSKLTVDKSRWQQGNV FSCSVMHEALHNHYTQKSLSLSPGK | |
| SEQ ID NO:73 | Chain 2: Fc (L234A/L235A/M252Y/S254T/T256E/P329G/Y349C/T366S/L368A/Y407V) Hole |
| DKTHTCPPCPAPEAAGGPSVFLFPPKPKDTLYITREPEVTCVVVDVSHEDPEVKFNWY VDGVEVHNAKTKPREEQYNSTYRVVSVLTVLHQDWLNGKEYKCKVSNKALGAPIEK TISKAKGQPREPQVCTLPPSREEMTKNQVSLSCAVKGFYPSDIAVEWESNGQPENNYK TTPPVLDSDGSFFLVSKLTVDKSRWQQGNVFSCSVMHEALHNHYTQKSLSLSPGK | |
| SEQ ID NO:64 | Chain 3: VL-CL (CD84) |
| BIS08 | |
| SEQ ID NO:74 | Chain 1: VH-CH-Fc (L234A/L235A/M252Y/S254T/T256E/P329G/S354C/T366W) (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | |
| SEQ ID NO:75 | Chain 2: Scfv-Fc (L234A/L235A/M252Y/S254T/T256E/P329G/Y349C/T366S/L368A/Y407V) (Hole) |
| EVQLVESGGGLVQPGGSLRLSCAASGFTFNTYAMNWVRQAPGKGLEWVARIRSKYN NYATYYAASVKGRFTISRDDSKNSLYLQMNSLKTEDTAVYYCARHGNFGDSYVSWFA YWGQGTLVTVSSGGGGSGGGGSGGGGSQTVVTQEPSLTVSPGGTVTLTCRSSTGAVT TSNYANWVQQKPGQAPRGLIGGTNKRAPGTPARFSGSLLGGKAALTLSGVQPEDEAE YYCALWYSNHWVFGGGTKLTVLDKTHTCPPCPAPEAAGGPSVFLFPPKPKDTLYITRE | |
| PEVTCVVVDVSHEDPEVKFNWYVDGVEVHNAKTKPREEQYNSTYRVVSVLTVLHQD WLNGKEYKCKVSNKALGAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCSV MHEALHNHYTQKSLSLSPGK | |
| SEQ ID NO:64 | Chain 3: VL-CL (CD84) |
| BIS09 | |
| SEQ ID NO:74 | Chain 1: VH-CH-Fc (L234A/L235A/M252Y/S254T/T256E/P329G/S354C/T366W) (Knob) |
| EVQLVQSGAEVKKPGATVKISCKASGYTFTDYYMKWVKQAHGKGLEWMGDINPNN GYTIYNQKFRGKVTITADKSTDTAYMELSSLRSEDTAVYYCARDVDGHDEGYFDYW GQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPEAAGGPSVFLFPPKPKDTLYITREPEVTCVVVDVSHEDPEVKFNWYVDG VEVHNAKTKPREEQYNSTYRVVSVLTVLHQDWLNGKEYKCKVSNKALGAPIEKTISK AKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | |
| SEQ ID NO:64 | Chain 2: VL-CL (CD84) |
| SEQ ID NO:76 | Chain 3: VH-CL-Fc (L234A/L235A/M252Y/S254T/T256E/P329G/Y349C/T366S/L368A/Y407V) (Hole) |
| EVQLVESGGGLVQPGGSLRLSCAASGFTFNTYAMNWVRQAPGKGLEWVARIRSKYN NYATYYAASVKGRFTISRDDSKNSLYLQMNSLKTEDTAVYYCARHGNFGDSYVSWFA YWGQGTLVTVSSASVAAPSVFIFPPSDEQLKSGTASVVCLLNNFYPREAKVQWKVDN ALQSGNSQESVTEQDSKDSTYSLSSTLTLSKADYEKHKVYACEVTHQGLSSPVTKSFN RGECDKTHTCPPCPAPEAAGGPSVFLFPPKPKDTLYITREPEVTCVVVDVSHEDPEVKF NWYVDGVEVHNAKTKPREEQYNSTYRVVSVLTVLHQDWLNGKEYKCKVSNKALG APIEKTISKAKGQPREPQVCTLPPSREEMTKNQVSLSCAVKGFYPSDIAVEWESNGQPE NNYKTTPPVLDSDGSFFLVSKLTVDKSRWQQGNVFSCSVMHEALHNHYTQKSLSLSP GK | |
| SEQ ID NO:77 | Chain 4: VL-CH (CD3) |
| QTVVTQEPSLTVSPGGTVTLTCRSSTGAVTTSNYANWVQQKPGQAPRGLIGGTNKRA PGTPARFSGSLLGGKAALTLSGVQPEDEAEYYCALWYSNHWVFGGGTKLTVLASTKG PSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSC | |

### Example 7 In Vitro T-Cell Dependent Cytotoxicity of Bispecific Antibodies

U937, a human histiocytic lymphoma cell and an acute myeloid leukemia cell, was used as the target cell, and human PBMCs were used as effector cells. The efficacies of the CD84×CD3 bispecific antibodies in Example 6 on T-cell mediated cytotoxicity were measured and analyzed. The specific operations were as follows: RPMI-1640 medium containing 1% FBS was prepared as the killing assay medium. Frozen PBMC effector cells were thawed. The PBMC cells were transferred to a 15 mL centrifuge tube, centrifuged at 1500 rpm for 5 min, and the supernatant was discarded. The cells were washed twice with medium, resuspended, and counted with a cell counter. The effector cell density was adjusted to 1.25×10⁶ cells/mL, and then stored in a 37 °C, 5% CO₂ incubator for later use. 5 mL of medium was added to a 15 mL centrifuge tube, and frozen target cells were thawed in a water bath at 37 °C and then transferred into 5 mL medium, and centrifuged at 1500 rpm for 5 min. The supernatant was discarded, and the cells were washed twice with medium. The cells were resuspended, and counted with a cell counter. The target cell density was adjusted to 1.88×10⁵ cells/mL, and then stored in a 37 °C, 5% CO₂ incubator for later use.

Antibody dilution: The initial concentrations of the CD84×CD3 bispecific antibodies in Example 6 were 20 nM and 10 nM, followed by 5-fold dilutions to obtain a total of 10 different concentrations.

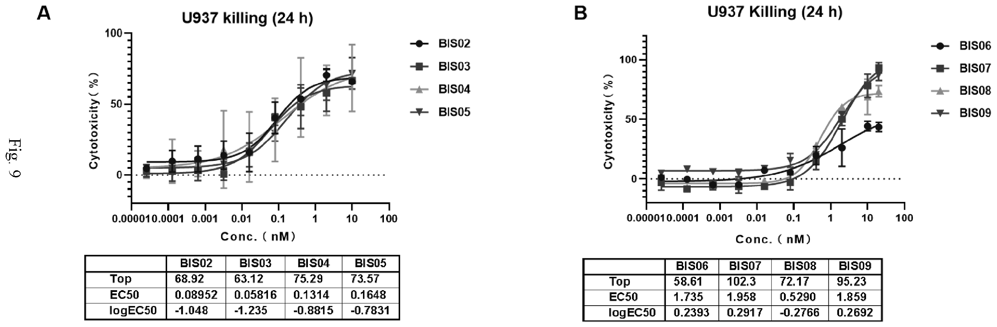

The reserved PBMC effector cells and target cells were taken out, and 60 µL/well of each was taken and added to a 96-well plate for culture. Using a multichannel pipette, 60 µL of the diluted antibodies was taken and added to the 96-well plate for incubation. The cell distribution was observed under a microscope, and 60 µL of medium was added to the control wells. It was placed in a 37 °C, 5% CO₂ incubator and cultured for 24 h. At 45 min before the end of co-culture, 20 µL of 10× lysis solution was added to the target cell maximum LDH release wells, mixed well, and the cells were cultured at 37 °C, 5% CO₂ for 45 min. The co-cultured cells were centrifuged at 3000 rpm for 2 min. 10 µL of the supernatant was transferred to a new 384-well microplate, and 10 µL of CytoTox96 reagent was added to each well, centrifuged at 1000 rpm for 1 min, shaken for 1 min, incubated at room temperature in the dark for 30 min, with the maximum reading controlled between 1 and 2. 10 µL of stop solution was added to each well and shaken for 1 min. The experimental results are shown in Fig. 9. The results indicate that bispecific antibody molecules BIS02, BIS03, BIS04 and BIS05 all exhibited potent PBMC-mediated killing effects against U937 cells, while BIS07, BIS08 and BIS09 exhibit certain activity.

### Example 8 Detection of Binding of Bispecific Antibodies to CD84 and CD3 Proteins

This example is an affinity assay of bispecific antibodies to CD84 and CD3 proteins, verifying whether the construction of the bispecific antibodies affects their binding affinity to CD84 and CD3 proteins.

The specific operations were as follows: 100 µL of 1 µg/mL CD84 or CD3 protein solution was added to each well of a 96-well microplate, and incubated overnight in a refrigerator at 2-8 °C. The liquid in each well was aspirated, and the plate was washed twice with washing buffer to remove excess antigen solution. After washing, the plate was inverted to remove residual liquid. At room temperature, 100 µL of 2% BSA (prepared in PBS buffer) was added to each well for blocking for 1 hour. The liquid in each well was aspirated, and the plate was washed twice with washing buffer to remove excess solution. After washing, the plate was inverted to remove residual liquid. 100 µL of the antibody solutions to be tested at gradient concentrations were added to each well and incubated at room temperature for 1 hour. The liquid in each well was aspirated, and the plate was washed five times with washing buffer. 100 µL of Goat Anti-Human IgG Secondary Antibody (HRP) (Sino Biological, SSA002) was added to each well and incubated at room temperature for 0.5 hour. The liquid in each well was aspirated, and the plate was washed five times with washing buffer. 100 µL of TMB substrate solution was added to each well, and the plate was incubated at room temperature for 10 minutes or longer until the solution reached the desired color intensity. 100 µL of HCl stop solution was added to each well. The absorbance of the solution in each well was measured at a wavelength of 450 nm using a microplate reader within 20 minutes after addition of the stop solution. BIS03, BIS05 and BIS07 were detected respectively, and CD3 mab, CD84 mab, isotype control (labeled as Isotype in the figs.) and PBS were used as controls.

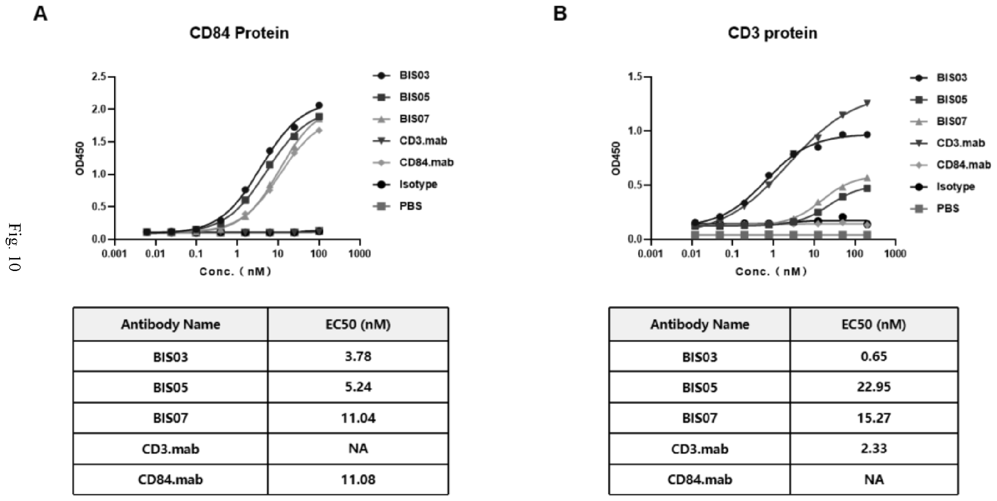

The experimental results are shown in Fig. 10, wherein the panel A is a graph of the binding assay results with CD84 protein, and the panel B is a graph of the binding assay results with CD3 protein. The experimental results indicate that the bispecific antibody configuration did not affect their binding to CD84 and CD3 proteins.

### Example 9 Detection of Binding of Bispecific Antibodies to Different Cells

Affinities of the bispecific antibodies to different cells (293T-hCD84, HEL and THP-1) were determined in this example to detect the binding ability of the bispecific antibody molecules to CD84 protein on different cell lines.

The specific operations were as follows: Pre-cultured cells (HEK293T-hCD84; HEL; THP-1) were digested and then resuspended in the FACS Buffer (PBS + 1% BSA) solution. After cell counting, cells were seeded into a 96-well V-bottom plate at the amount of 2E5 cells/100 µL per well. After cell treatment was completed, antibodies were added to each well in a volume of 100 µL at a preset concentration, mixed well with the cells, and then incubated at 4°C for 1 h, which were centrifuged at 1500 rpm/min for 5 min after the incubation. The supernatant was discarded, and the cell pellet was retained and the cells were resuspended by adding 200 µL of FACS buffer. Centrifugation and washing were repeated twice under the same conditions. Then, 100 µL of the secondary antibody Alexa Fluor^{®} 647-conjugated AffiniPure^{™} Goat Anti-Human IgG (Jackson 109-605-098) was added thereto, and incubation was continued for 0.5 h, followed by centrifugation and washing twice. Finally, the cells were resuspended by adding 100 µL of FACS Buffer and subjected to detection on an instrument. The number of cells per sample should not be less than 10,000 during detection. BIS03, BIS05 and BIS07 were detected respectively, and CD3 mab, CD84 mab and Isotype were used as controls.

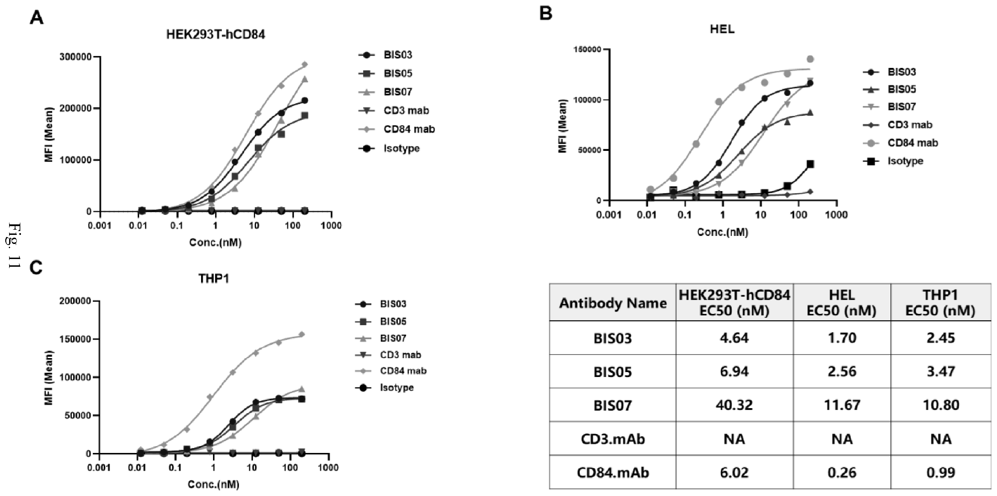

The experimental results are shown in Fig. 11, wherein A is a graph of the experimental results for HEK293T-hCD84 cells, B is a graph of the experimental results for HEL cells, and C is a graph of the experimental results for THP-1 cells, with the table showing the specific EC50 detection data. The experimental results show that BIS03, BIS05 and BIS07 all had strong binding ability to HEK293T-hCD84, HEL and THP-1 cell lines, indicating that the bispecific antibody molecules had strong binding ability to CD84 protein on overexpressing cells or tumor cells.

### Example 10 Bispecific Antibody-Mediated Killing of Immune Cells against Tumor Cells

This example was used to detect the bispecific antibody-mediated toxicity of immune cells against tumor cells (AML cells). The specific operations were as follows: Pre-cultured tumor cells (HEL; U937; THP-1; MOML-13) were trypsinized and then resuspended in the culture medium (PBS + 2% FBS) solution. After cell counting, cells were seeded into a 96-well cell culture plate at the amount of 1.5E4 cells/100 µL per well, and placed in a 37 °C incubator for 4-5 h. PBMCs were counted, and added to the cell culture plate at the amount of 1.5E5 cells/50 µL per well calculated according to a 10:1 ratio of PBMCs to tumor cells. 50 µL of the antibody was added to each well at a preset concentration, and incubated in a 37 °C incubator for 24 h. 150 µL of the supernatant was aspirated and added to a clean 96-well plate for subsequent ELISA and LDH assays. 50 µL of CTG (Promega, Cat. No.: G7573) was added to the remaining approximately 50 µL of the 96-well plate, gently tapped to mix well, and 80 µL of the reaction solution was aspirated and added to a white-edged 96-well plate with a transparent bottom, where luminescence data was detected by a microplate reader.

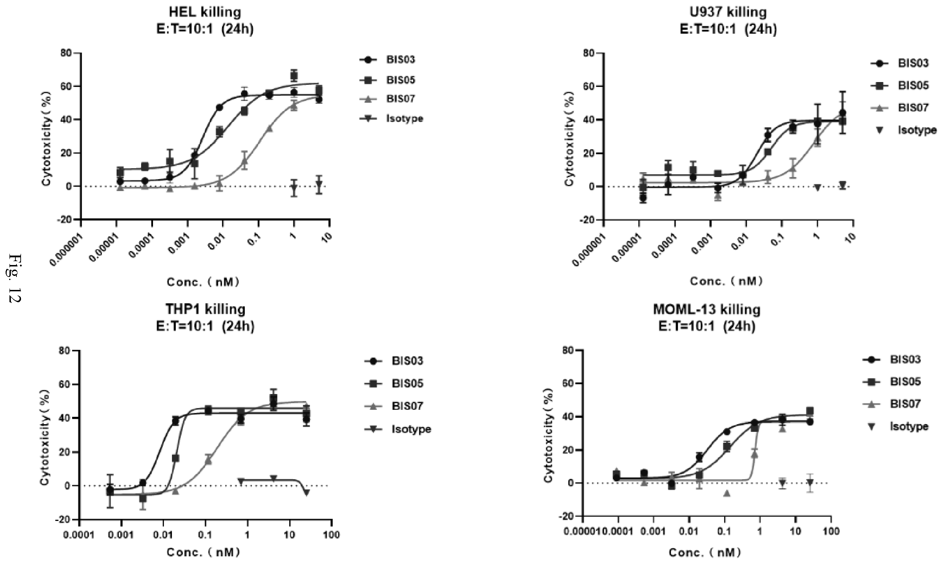

The experimental results are shown in Fig. 12. The experimental results indicate that bispecific antibodies could significantly enhance the toxicity of immune cells against tumor cells (AML cells).

### Example 11 In Vitro Safety Evaluation of Bispecific Antibodies

*In vitro* safety evaluation of the bispecific antibodies was performed in this example. The specific operations were as follows: The prepared PBMCs were resuspended in the culture medium (PBS + 2% FBS) solution and added to a cell culture plate at the amount of 22.5E5 cells/100 µL per well. Antibodies were added to each well in a volume of 100 µL at a preset concentration, incubated in a 37 °C incubator for 72 h. 150 µL of the supernatant was aspirated and added to a clean 96-well plate for subsequent ELISA and LDH assays. 50 µL of CTG (Promega, Cat. No.: G7573) was added to the remaining approximately 50 µL of the 96-well plate, gently tapped to mix well, and 80 µL of the reaction solution was aspirated and added to a white-edged 96-well plate with a transparent bottom, where luminescence data was detected by a microplate reader. BIS03, BIS05 and BIS07 were detected respectively, and CD84 mab, Talquetamab and Isotype were used as controls. Talquetamab is a CD3/GPRC5D bispecific T cell engaging antibody.

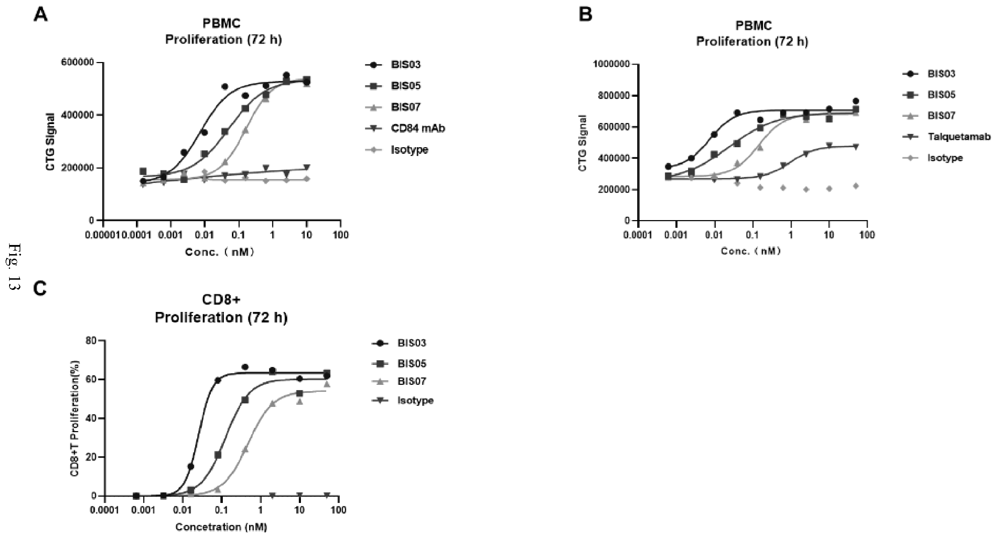

The experimental results are shown in Fig. 13. The experimental results indicate that all the bispecific antibody molecules could significantly stimulate the proliferation and activation of PBMCs *in vitro*, with a certain potential risk of a cytokine storm. The proliferation effect of CD8⁺ T cells during PBMC proliferation has little effect on the self-killing of T cells.

### Example 12 Kinetics Assay of Bispecific Antibodies with CD84 and CD3 Proteins

The kinetics of bispecific antibodies to CD84 and CD3 proteins were detected in this example. The specific operations were as follows: The binding kinetics (KD) of the two bispecific antibody molecules to CD3 and CD84 proteins were detected by a Gator^{™} label-free biomolecular analyzer using Bio-Layer Interferometry (BLI) technique. CD3 and CD84 proteins were diluted to 1 µg/mL and immobilized on a his biosensor. The antibodies were then diluted to 50 nM, 25 nM, 12.5 nM, 6.25 nM, 3.125 nM, and 1.5625 nM, respectively, to bind to the proteins at a total of 6 concentration gradients. The results were fitted using a 1:1 model to calculate and obtain the equilibrium dissociation constant (KD) values of the samples.

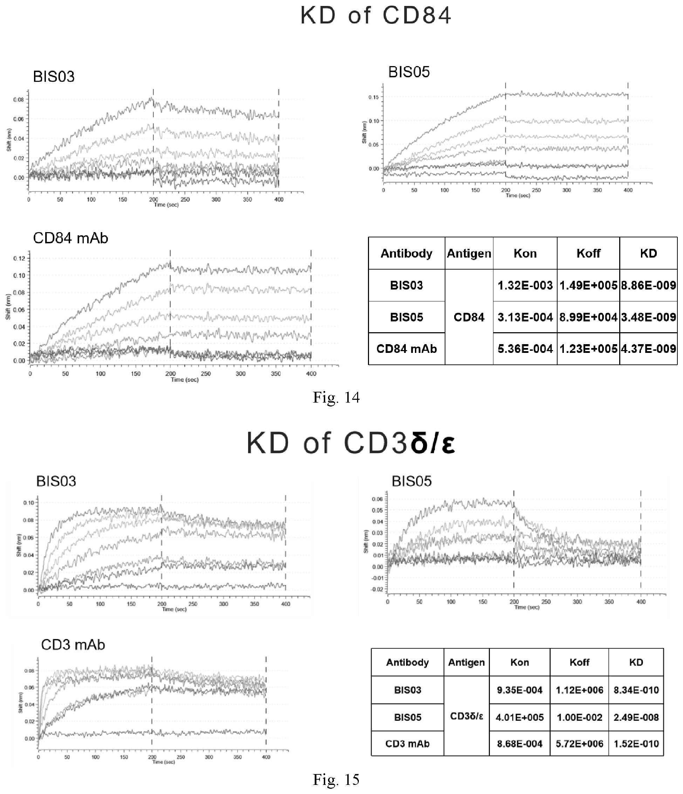

The experimental results are shown in Fig. 14 and Fig. 15. Fig. 14 is the kinetics experimental results of the bispecific antibody molecules with CD84 protein, and Fig. 15 is the kinetics experimental results of CD3 protein. The experimental results measured the KD values of BIS03 and BIS05 to CD84 and CD3, demonstrating that the bispecific antibody molecules possessed strong affinity for both CD84 and CD3.

### Example 13 In Vivo Anti-Tumor Assay of Bispecific Antibodies

This example is an *in vivo* anti-tumor assay of the bispecific antibodies. The specific operations were as follows: (1) inoculation and modeling of an orthotopic tumor via tail vein: Leukemia cells THP1-luciferase (AML cell line) with good growth status were taken, washed once with sterile PBS after centrifugation, centrifuged again, resuspended and counted. The cell density was adjusted with sterile PBS. THP1-luciferase cells were transplanted via tail vein into mice (6-week old female NSG mice, acclimated for one week after arrival at the animal room). After the mouse tail was disinfected with 75% alcohol, 1×10⁶ cells (200 µL) of cell suspension were injected via tail vein using a 1 mL syringe, which was recorded as day 0. (2) PBMC was given to mice for treatment: Five days after tumor cell transplantation of mice, PBMCs were injected via tail vein according to E:T = 10:1. All mice were then randomly divided into three groups according to body weight: Control, BIS03 and BIS05 groups, with 8 mice in each group. Administration was performed via tail vein injection on day 6, day 9, day 13, day 16, day 20 and day 23, respectively, at a dose of 10 µg per mouse (0.5 mpk). Imaging observation was performed on day 7, day 14, day 21 and day 28. The total experimental period was 77 days. (3) Mouse body weight was measured weekly to plot mouse body weight curve; mouse bioluminescence imaging was performed weekly to monitor tumor burden in mice; survival time of mice was recorded to plot the survival curve.

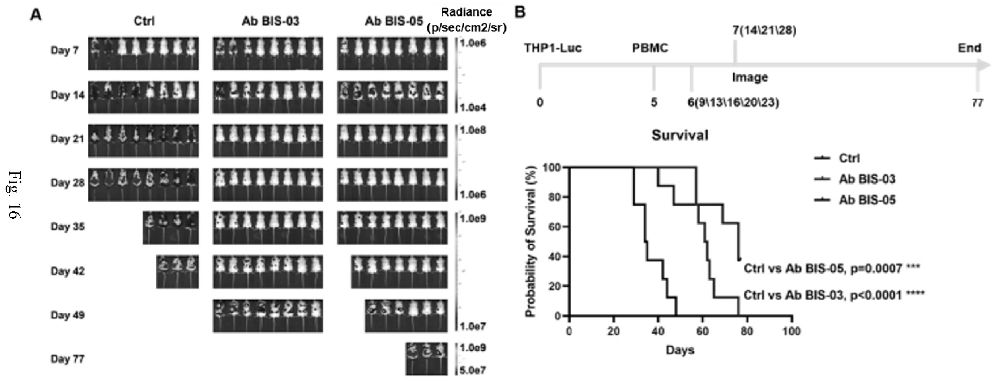

The experimental results are shown in Fig. 16. The experimental results show that the survival time of mice was significantly prolonged, demonstrating that the bispecific antibody molecules exhibited significant *in vivo* anti-tumor effects.

## Claims

1. An antibody or antigen-binding fragment thereof that binds to CD84, comprising three complementarity-determining regions of a heavy chain variable region (HCDRs)and three complementarity-determining regions of a light chain variable region (LCDRs), wherein:
(i) HCDR1 comprises or consists of the amino acid sequence of SEQ ID NO: 1, HCDR2 comprises or consists of the amino acid sequence of SEQ ID NO: 2, HCDR3 comprises or consists of the amino acid sequence of SEQ ID NO: 3, LCDR1 comprises or consists of the amino acid sequence of SEQ ID NO: 4, LCDR2 comprises or consists of NAK, and LCDR3 comprises or consists of the amino acid sequence of SEQ ID NO: 5; or
(ii) HCDR1 comprises or consists of the amino acid sequence of SEQ ID NO: 1, HCDR2 comprises or consists of the amino acid sequence of SEQ ID NO: 2, HCDR3 comprises or consists of the amino acid sequence of SEQ ID NO: 3, LCDR1 comprises or consists of the amino acid sequence of SEQ ID NO: 6, LCDR2 comprises or consists of FAS, and LCDR3 comprises or consists of the amino acid sequence of SEQ ID NO: 7; or
(iii) HCDR1 comprises or consists of the amino acid sequence of SEQ ID NO: 8, HCDR2 comprises or consists of the amino acid sequence of SEQ ID NO: 9, HCDR3 comprises or consists of the amino acid sequence of SEQ ID NO: 10, LCDR1 comprises or consists of the amino acid sequence of SEQ ID NO: 11, LCDR2 comprises or consists of WAS, and LCDR3 comprises or consists of the amino acid sequence of SEQ ID NO: 12; or
(iv) HCDR1 comprises or consists of the amino acid sequence of SEQ ID NO: 13, HCDR2 comprises or consists of the amino acid sequence of SEQ ID NO: 14, HCDR3 comprises or consists of the amino acid sequence of SEQ ID NO: 15, LCDR1 comprises or consists of the amino acid sequence of SEQ ID NO: 16, LCDR2 comprises or consists of YAS, and LCDR3 comprises or consists of the amino acid sequence of SEQ ID NO: 17; or
(v) HCDR1 comprises or consists of the amino acid sequence of SEQ ID NO: 8, HCDR2 comprises or consists of the amino acid sequence of SEQ ID NO: 18, HCDR3 comprises or consists of the amino acid sequence of SEQ ID NO: 10, LCDR1 comprises or consists of the amino acid sequence of SEQ ID NO: 19, LCDR2 comprises or consists of WAS, and LCDR3 comprises or consists of the amino acid sequence of SEQ ID NO: 20; or
(vi) HCDR1 comprises or consists of the amino acid sequence of SEQ ID NO: 8, HCDR2 comprises or consists of the amino acid sequence of SEQ ID NO: 9, HCDR3 comprises or consists of the amino acid sequence of SEQ ID NO: 21, LCDR1 comprises or consists of the amino acid sequence of SEQ ID NO: 11, LCDR2 comprises or consists of WAS, and LCDR3 comprises or consists of the amino acid sequence of SEQ ID NO: 12; or
(vii) HCDR1 comprises or consists of the amino acid sequence of SEQ ID NO: 22, HCDR2 comprises or consists of the amino acid sequence of SEQ ID NO: 23, HCDR3 comprises or consists of the amino acid sequence of SEQ ID NO: 24, LCDR1 comprises or consists of the amino acid sequence of SEQ ID NO: 25, LCDR2 comprises or consists of WAS, and LCDR3 comprises or consists of the amino acid sequence of SEQ ID NO: 26; or
(viii) HCDR1 comprises or consists of the amino acid sequence of SEQ ID NO: 22, HCDR2 comprises or consists of the amino acid sequence of SEQ ID NO: 23, HCDR3 comprises or consists of the amino acid sequence of SEQ ID NO: 24, LCDR1 comprises or consists of the amino acid sequence of SEQ ID NO: 27, LCDR2 comprises or consists of WAS, and LCDR3 comprises or consists of the amino acid sequence of SEQ ID NO: 26;
or wherein the antibody comprises a variant of one of the CDR sequence combinations (i) - (viii),
wherein the variant comprises a total of at least one and not more than 5, 4, 3, 2 or 1 amino acid alterations (preferably amino acid substitutions, more preferably conservative substitutions) in the six CDR regions, and preferably the heavy chain CDR3 remains unchanged;
wherein the amino acid X at position 5 in the amino acid sequence of SEQ ID NO: 9 is N, D, or Q.

2. The antibody or antigen-binding fragment thereof according to claim 1, comprising a heavy chain variable region and a light chain variable region, wherein:
the heavy chain variable region comprises:
(i) the amino acid sequence set forth in SEQ ID NO: 30, or an amino acid sequence having at least 80%, 85%, 90%, 95%, 96%, 97%, 98% or 99% sequence identity thereto; or
(ii) the amino acid sequence set forth in SEQ ID NO: 43, or an amino acid sequence having at least 80%, 85%, 90%, 95%, 96%, 97%, 98% or 99% sequence identity thereto; or
(iii) the amino acid sequence set forth in SEQ ID NO: 45, or an amino acid sequence having at least 80%, 85%, 90%, 95%, 96%, 97%, 98% or 99% sequence identity thereto; or
(iv) the amino acid sequence set forth in SEQ ID NO: 53, or an amino acid sequence having at least 80%, 85%, 90%, 95%, 96%, 97%, 98% or 99% sequence identity thereto.

3. The antibody or antigen-binding fragment thereof according to claim 1 or 2, comprising a heavy chain variable region and a light chain variable region, wherein:
the light chain variable region comprises:
(i) the amino acid sequence set forth in SEQ ID NO: 29, or an amino acid sequence having at least 80%, 85%, 90%, 95%, 96%, 97%, 98% or 99% sequence identity thereto; or
(ii) the amino acid sequence set forth in SEQ ID NO: 38, or an amino acid sequence having at least 80%, 85%, 90%, 95%, 96%, 97%, 98% or 99% sequence identity thereto; or
(iii) the amino acid sequence set forth in SEQ ID NO: 46, or an amino acid sequence having at least 80%, 85%, 90%, 95%, 96%, 97%, 98% or 99% sequence identity thereto; or
(iv) the amino acid sequence set forth in SEQ ID NO: 54, or an amino acid sequence having at least 80%, 85%, 90%, 95%, 96%, 97%, 98% or 99% sequence identity thereto.

4. The antibody or antigen-binding fragment thereof according to any one of claims 1 to 3, comprising a heavy chain variable region and a light chain variable region selected from:
(i) a heavy chain variable region comprising the amino acid sequence set forth in SEQ ID NO: 30, and a light chain variable region comprising the amino acid sequence set forth in SEQ ID NO: 29; or
(ii) a heavy chain variable region comprising the amino acid sequence set forth in SEQ ID NO: 43, and a light chain variable region comprising the amino acid sequence set forth in SEQ ID NO: 38; or
(iii) a heavy chain variable region comprising the amino acid sequence set forth in SEQ ID NO: 45, and a light chain variable region comprising the amino acid sequence set forth in SEQ ID NO: 46; and
(iv) a heavy chain variable region comprising the amino acid sequence set forth in SEQ ID NO: 53, and a light chain variable region comprising the amino acid sequence set forth in SEQ ID NO: 54.

5. The antibody or antigen-binding fragment thereof according to any one of claims 1 to 4, wherein the antibody is an antibody or antigen-binding fragment thereof in the form of IgG1, IgG2, IgG3, or IgG4, preferably wherein the antibody comprises a human IgG1 Fc region.

6. The antibody or antigen-binding fragment thereof according to any one of claims 1 to 5, wherein the antibody is a murine antibody, or chimeric antibody, or humanized antibody, or wherein the antigen-binding fragment is an antibody fragment selected from Fab, Fab', Fab'-SH, Fv, single-chain antibody, scFv, (Fab')2 fragment, single-domain antibody, diabody (dAb), and linear antibody.

7. The antibody or antigen-binding fragment thereof according to any one of claims 1 to 6, wherein the antibody is a multispecific antibody.

8. The antibody or antigen-binding fragment thereof according to claim 7, wherein the antibody is a bispecific antibody which further comprises an antigen-binding fragment that binds to a second antigen.

9. The antibody or antigen-binding fragment thereof according to claim 8, wherein the second antigen is a tumor-associated antigen or an immune cell antigen, preferably a T cell antigen.

10. The antibody or antigen-binding fragment thereof according to claim 9, wherein the T cell antigen is selected from T cell receptor (TCR), CD3, CD4, CD8, CD16, CD25, CD28, CD38, CD44, CD62L, CD69, ICOS, 41-BB (CD137), and NKG2D.

11. A bispecific antibody or antigen-binding fragment thereof, comprising a first antigen-binding fragment that binds to CD84 and a second antigen-binding fragment that binds to CD3, wherein the first antigen-binding fragment comprises a first light chain variable region (VL1) and a first heavy chain variable region (VH1), the second antigen-binding fragment comprises a second light chain variable region (VL2) and a second heavy chain variable region (VH2), wherein the VL1 comprises LCDRs 1-3 of the amino acid sequence set forth in SEQ ID NO: 29, 38, 46 or 54, and the VH1 comprises HCDRs 1-3 of the amino acid sequence set forth in SEQ ID NO: 30, 43, 45 or 53; and/or the VL2 comprises LCDRs 1-3 of the amino acid sequence set forth in SEQ ID NO: 62, and the VH2 comprises HCDRs 1-3 of the amino acid sequence set forth in SEQ ID NO: 61.

12. The bispecific antibody according to any one of claims 8 to 11, wherein the second antigen-binding region is a scFv comprising VL2 and VH2, and the scFv is optionally linked to the N-/C-terminus of the VL1 or VH1 or to the N-/C-terminus of Fc via a linker.

13. The bispecific antibody according to claim 12, wherein the linker comprises an amino acid sequence selected from (G4S)n and GS(G4S)n, wherein n is an integer selected from 1 to 5.

14. An isolated nucleic acid, encoding the anti-CD84 antibody or antigen-binding fragment thereof according to any one of the preceding claims.

15. A vector, comprising the nucleic acid according to claim 14, preferably the vector is an expression vector.

16. A host cell, comprising the nucleic acid according to claim 14 or the vector according to claim 15, preferably the host cell is a mammalian cell.

17. A method for preparing an anti-CD84 antibody or antigen-binding fragment thereof, comprising: culturing a host cell comprising the nucleic acid under conditions that expression of the antibody or antigen-binding fragment thereof according to any one of claims 1 to 13, optionally isolating the antibody or antigen-binding fragment thereof, and optionally recovering the anti-CD84 antibody or antigen-binding fragment thereof from the host cell.

18. An immunoconjugate, comprising the anti-CD84 antibody or antigen-binding fragment thereof according to any one of the preceding claims 1 to 13, conjugated to a therapeutic agent or a diagnostic agent.

19. A pharmaceutical composition, comprising the antibody or antigen-binding fragment thereof according to any one of the preceding claims 1 to 13 or the immunoconjugate according to claim 18, and optionally a pharmaceutical excipient.

20. A method of preventing or treating a tumor in a subject, comprising administering to the subject an effective amount of the anti-CD84 antibody or antigen-binding fragment thereof according to any one of the preceding claims 1 to 13, or the immunoconjugate according to claim 18, or the pharmaceutical composition according to claim 19, preferably the tumor is a hematological malignancy; preferably the hematological malignancy is acute myeloid leukemia (AML), chronic lymphocytic leukemia (CLL), blastic plasmacytoid dendritic cell neoplasm (BPDCN), chronic myeloid leukemia (CML), myelodysplastic syndrome (MDS), acute lymphoblastic leukemia (ALL), multiple myeloma (MM), hairy cell leukemia (HCL), or Hodgkin's lymphoma (HL).

21. A method for detecting CD84 in a sample, comprising:
(a) contacting the sample with the antibody or antigen-binding fragment thereof according to any one of the preceding claims 1 to 13; and
(b) detecting the formation of a complex between the antibody or antigen-binding fragment thereof and CD84; optionally, the antibody is detectably labeled.
